# EUROPEAN PATENT APPLICATION

(11) **EP 2 356 999 A1**
(43) Date of publication of application: **17.08.2011**
(21) Application number: 10180566.1
(22) Date of filing: 17.06.2004
(51) Int. Cl.: A61K 39/00, A61P 35/00, A61P 37/00

(54) **Compositions to elicit, enhance and sustain immune responses against MHC class I-restricted epitopes, for prophylactic or therapeutic purposes**

(30) Priority: 17.06.2003 US 479393 P
(62) Divisional of application: 10171754.4
(71) Applicant: Mannkind Corporation, Valencia, California 91355 (US)
(72) Inventor: Bot, Adrian Ion, Valencia, CA 91354 (US); Liu, Xiping, Temple City, CA 91780 (US); Smith, Kent Andrew, Ventura, CA 93001 (US)
(74) Representative: Lasar, Andrea Gisela

(57) **Abstract**

Embodiments relate to methods and compositions for eliciting, enhancing, and sustaining immune responses, preferably against MHC class I-restricted epitopes. The methods and compositions can be used for prophylactic or therapeutic purposes.

## Description

### Background of the Invention

### Field of the Invention

The invention disclosed herein relates to methods and compositions for inducing a MHC class I-restricted immune response and controlling the nature and magnitude of the response, promoting effective immunologic intervention in pathogenic processes. More particularly it relates to immunogenic compositions, their nature and the order, timing, and route of administration by which they are effectively used.

### Description of the Related Art

### The Major Histocompatibility Complex and T Cell Target Precognition

T lymphocytes (T cells) are antigen-specific immune cells that function in response to specific antigen signals. B lymphocytes and the antibodies they produce are also antigen-specific entities. However, unlike B lymphocytes, T cells do not respond to antigens in a free or soluble form. For a T cell to respond to an antigen, it requires the antigen to be bound to a presenting complex known as the major histocompatibility complex (MHC).

MHC proteins provide the means by which T cells differentiate native or "self" cells from foreign cells. MHC molecules are a category of immune receptors that present potential peptide epitopes to be monitored subsequently by the T cells There are two types of MHC, class I MHC and class II MHC. CD4⁺ T cells interact with class II MHC proteins and predominately have a helper phenotype while CD8⁺ T cells interact with class I MHC proteins and predominately have a cytolytic phenotype, but each of them can also exhibit regulatory, particularly suppressive, function. Both MHC are transmembrane proteins with a majority of their structure on the external surface of the cell. Additionally, both classes of MHC have a peptide binding cleft on their external portions. It is in this cleft that small fragments of proteins, native or foreign, are bound and presented to the extracellular environment.

Cells called antigen presenting cells (APCs) display antigens to T cells using the MHC. T cells can recognize an antigen, if it is presented on the MHC. This requirement is called MHC restriction. If an antigen is not displayed by a recognizable MHC, the T cell will not recognize and act on the antigen signal. T cells specific for the peptide bound to a recognizable MHC bind to these MHC-peptide complexes and proceed to the next stages of the immune response.

Peptides corresponding to nominal MHC class I or class II restricted epitopes are among the simplest forms of antigen that can be delivered for the purpose of inducing, amplifying or otherwise manipulating the T cell response. Despite the fact that peptide epitopes have been shown to be effective *in vitro* at re-stimulating *in vivo* primed T cell lines, clones, or T cell hybridomas, their *in vivo* efficacy has been very limited. This is due to two main factors:
(1) The poor pharmacokinetic (PK) profile of peptides, caused by rapid renal clearance and/or *in vivo* degradation, resulting in limited access to APC;
(2) The insufficiency of antigen-induced T cell receptor (TCR)-dependent signaling alone (signal 1) to induce or amplify a strong and sustained immune response, and particularly a response consisting of Tc1 or Th1 cells (producing IFN-y and TNF-alpha). Moreover, use of large doses of peptide or depot adjuvants, in order to circumvent the limited PK associated with peptides, can trigger a variable degree of unresponsiveness or "immune deviation" unless certain immune potentiating or modulating adjuvants are used in conjunction.

### Summary of the Invention

Embodiments of the present invention include methods and compositions for manipulating, and in particular for inducing, entraining, and/or amplifying, the immune response to MHC class I restricted epitopes.

Some embodiments relate to methods of immunization. The methods can include, for example, delivering to a mammal a first composition that includes an immunogen, the immunogen can include or encode at least a portion of a first antigen; and admincan betering a second composition, which can include an amplifying peptide, directly to a lymphatic system of the mammal, wherein the peptide corresponds to an epitope of said first antigen, wherein the first composition and the second composition are not the same. The methods can further include the step of obtaining, assaying for or detecting and effector T cell response.

The first composition can include a nucleic acid encoding the antigen or an immunogenic fragment thereof. The first composition can include a nucleic acid capable of expressing the epitope in a pAPC. The the nucleic acid can be delivered as a component of a protozoan, bacterium, virus, or viral vector. The first composition can include an immunogenic polypeptide and an immunopotentiator, for example. The immunopotentiator can be a cytokine, a toll-like receptor ligand, and the like. The adjuvant can include an immunostimulatory sequence, an RNA, and the like.

The immunogenic polypeptide can be said amplifying peptide. The immunogenic polypeptide can be said first antigen. The immunogenic polypeptide can be delivered as a component of a protozoan, bacterium, virus, viral vector, or virus-like particle, or the like. The adjuvant can be delivered as a component of a protozoan, bacterium, virus, viral vector, or virus-like particle, or the like. The second composition can be adjuvant-free and immunopotentiator-free. The delivering step can include direct administration to the lymphatic system of the mammal. The direct administration to the lymphatic system of the mammal can include direct administration to a lymph node or lymph vessel. The direct administration can be to two or more lymph nodes or lymph vessels. The lymph node can be, for example, inguinal, axillary, cervical, and tonsilar lymph nodes. The effector T cell response can be a cytotoxic T cell response. The effector T cell response can include production of a pro-inflammatory cytokine, and the cytokine can be, for example, γ-IFN or TNFα. The effector T cell response can include production of a T cell chemokine, for example, RANTES or MIP-1α, or the like.

The epitope can be a housekeeping epitope or an immune epitope, for example. The delivering step or the administering step can include a single bolus injection, repeated bolus injections, for example. The delivering step or the administering step can include a continuous infusion, which for example, can have duration of between about 8 to about 7 days. The method can include an interval between termination of the delivering step and beginning the administering step, wherein the interval can be at least about seven days. Also, the interval can be between about 7 and about 14 days, about 17 days, about 20 days, about 25 days, about 30 days, about 40 days, about 50 days, or about 60 days, for example. The interval can be over about 75 days, about 80 days, about 90 days, about 100 days or more.

The first antigen can be a disease-associated antigen, and the disease-associated antigen can be a tumor-associated antigen, a pathogen-associated antigen. Embodiments include methods of treating disease utilizing the described method of immunizing. The first antigen can be a target-associated antigen. The target can be a neoplastic cell, a pathogen-infected cell, and the like. For example, any neoplastic cell can be targeted. Pathogen-infected cells can include, for example, cells infected by a bacterium, a virus, a protozoa, a fungi, and the like, or affected by a prion, for example.

The effector T cell response can be detected by at least one indicator for example, a cytokine assay, an Elcan bepot assay, a cytotoxicity assay, a tetramer assay, a DTH-response, a clinical response, tumor shrinkage, tumor clearance, inhibition of tumor progression, decrease pathogen titre, pathogen clearance, amelioration of a disease symptom, and the like. The methods can further include obtaining, detecting or assaying for an effector T cell response to the first antigen.

Further embodiments relate to methods of immunization that include delivering to a mammal a first composition including a nucleic acid encoding a first antigen or an immunogenic fragment thereof; administering a second composition, including a peptide, directly to the lymphatic system of the mammal, wherein the peptide corresponds to an epitope of said first antigen. The methods can further include obtaining, detecting or assaying for an effector T cell response to the antigen.

Also, embodiments relate to methods of augmenting an existing antigen-specific immune response. The methods can include administering a composition, that includes a peptide, directly to the lymphatic system of a mammal, wherein the peptide corresponds to an epitope of said antigen, and wherein said composition was not used to induce the immune response. The methods can further include obtaining, detecting or assaying for augmentation of an antigen-specific immune response. The augmentation can include sustaining the response over time, reactivating quiescent T cells, expanding the population of antigen-specific T cells, and the like. In some aspects, the composition may not include an immunopotentiator.

Other embodiments relate to methods of immunization which can include delivering to a mammal a first composition comprising an immunogen, the immunogen can include or encode at least a portion of a first antigen and at least a portion of a second antigen; administering a second composition including a first peptide, and a third composition including a second peptide, directly to the lymphatic system of the mammal, wherein the first peptide corresponds to an epitope of said first antigen, and wherein the second peptide corresponds to an epitope of said second antigen, wherein the first composition can be not the same as the second or third compositions. The methods further can include obtaining, detecting or assaying for an effector T cell response to said first and second antigens. The second and third compositions each can include the first and the second peptides. The second and third compositions can be part of a single composition.

Still further embodiments relate to methods of generating an antigen-specific tolerogenic or regulatory immune response. The methods can include periodically administering a composition, including an adjuvant-free peptide, directly to the lymphatic system of a mammal, wherein the peptide corresponds to an epitope of said antigen, and wherein the mammal can be epitopically naïve. The methods further can include obtaining, detecting and assaying for a tolerogenic or regulatory T cell immune response. The immune response can assist in treating an inflammatory disorder, for example. The inflammatory disorder can be, for example, from a class II MHC-restricted immune response. The immune response can include production of an immunosuppressive cytokine, for example, IL-5, IL-10, or TGB-β, and the like.

Embodiments relate to methods of immunization that include administering a series of immunogenic doses directly into the lymphatic system of a mammal wherein the series can include at least 1 entraining dose and at least 1 amplifying dose, and wherein the entraining dose can include a nucleic acid encoding an immunogen and wherein the amplifying dose can be free of any virus, viral vector, or replication-competent vector. The methods can further include obtaining an antigen-specific immune response. The methods can include, for example, 1-6 entraining doses. The method can include administering a plurality of entraining doses, wherein said doses are administered over a course of one to about seven days. The entraining doses, amplifying doses, or entraining and amplifying doses can be delivered in multiple pairs of injections, wherein a first member of a pair can be administered within about 4 days of a second member of the pair, and wherein an interval between first members of different pairs can be at least about 14 days. An interval between a last entraining dose and a first amplifying dose can be between about 7 and about 100 days, for example.

Other embodiments relate to sets of immunogenic compositions for inducing an immune response in a mammal including 1-6 entraining doses and at least one amplifying dose, wherein the entraining doses can include a nucleic acid encoding an immunogen, and wherein the amplifying dose can include a peptide epitope, and wherein the epitope can be presented by pAPC expressing the nucleic acid. The one dose further can include an adjuvant, for example, RNA. The entraining and amplifying doses can be in a carrier suitable for direct administration to the lymphatic system, a lymph node and the like. The nucleic acid can be a plasmid. The epitope can be a class I HLA epitope, for example, one listed in Tables 1-4. The HLA preferably can be HLA-A2. The immunogen can include an epitope array, which array can include a liberation sequence. The immunogen can consist essentially of a target-associated antigen. The target-associated antigen can be a tumor-associated antigen, a microbial antigen, any other antigen, and the like. The immunogen can include a fragment of a target-associated antigen that can include an epitope cluster.

Further embodiments can include sets of immunogenic compositions for inducing a class I MHC-restricted immune response in a mammal including 1-6 entraining doses and at least one amplifying dose, wherein the entraining doses can include an immunogen or a nucleic acid encoding an immunogen and an immunopotentiator, and wherein the amplifying dose can include a peptide epitope, and wherein the epitope can be presented by pAPC. The nucleic acid encoding the immunogen further can include an immunostimulatory sequence which serves as the immunopotentiating agent. The immunogen can be a virus or replication-competent vector that can include or can induce an immunopotentiating agent. The immunogen can be a bacterium, bacterial lysate, or purified cell wall component. Also, the bacterial cell wall component can serve as the immunopotentiating agent. The immunopotentiating agent can be, for example, a TLR ligand, an immunostimulatory sequence, a CpG-containing DNA, a dsRNA, an endocytic-Pattern Recognition Receptor (PRR) ligand, an LPS, a quillaja saponin, tucaresol, a pro-inflammatory cytokine, and the like.

Other embodiments relate to methods of generating various cytokine profiles. In some embodiments of the instant invention, intranodal administration of peptide can be effective in amplifying a response initially induced with a plasmid DNA vaccine. Moreover, the cytokine profile can be distinct, with plasmid DNA induction/peptide amplification generally resulting in greater chemokine (chemoattractant cytokine) and lesser immunosuppressive cytokine production than either DNA/DNA or peptide/peptide protocols.

Still further embodiments relate to uses of a peptide in the manufacture of an adjuvant-free medicament for use in an entrain-and-amplify immunization protocol. The compositions, kits, immunogens and compounds can be used in medicaments for the treatment of various diseases, to amplify immune reponses, to generate particular cytokine profiles, and the like, as described herein. Embodiments relate to the use of adjuvant-free peptide in a method of amplifying an immune response.

Embodiments are directed to methods, uses, therapies and compositions related to epitopes with specificity for MHC, including, for example, those listed in Tables 1-4. Other embodiments include one or more of the MHCs listed in Tables 1-4, including combinations of the same, while other embodiments specifically exclude any one or more of the MHCs or combinations thereof. Tables 3-4 include frequencies for the listed HLA antigens.

### Brief Description of the Drawings

Fig. 1 A-C: Induction of immune responses by intra-lymphatic immunization.

Figure 2 depicts examples of protocols for controlling or manipulating the immunity to MHC class I-restricted epitopes by targeted (lymph node) delivery of antigen.

Figure 3 represents a visual perspective on representative wells corresponding to the data described in Figure 4.

Figure 4 depicts the magnitude of immune response resulting from application of protocols described in Figure 2, measured by ELISPOT and expressed as number (frequency) of IFN-γ (gamma) producing T cells recognizing the peptide

Figure 5 shows the cytotoxic profile of T cells generated by targeted delivery of antigen, as described in Figure 2.

Figure 6 depicts the cross-reactivity of MHC class I-restricted T cells generated by the protocol depicted in the Figure 2.

Figure 7A shows the profile of immunity, expressed as ability of lymphocytes to produce members of three classes of biological response modifiers (pro-inflammatory cytokines, chemokines or chemo-attractants, and immune regulatory or suppressor cytokines), subsequent to application of the immunization protocols described in the Figure 2.

Figure 7B shows cell surface marker phenotyping by flow cytometry for T cell generated by the immunization protocols described in Figure 2. Repeated administration of peptide to the lymph nodes induces immune deviation and regulatory T cells.

Figure 8A and B show the frequency of specific T cells measured by tetramer, in mice immunized with DNA, peptide or an entrain/amplify sequence of DNA and peptide.

Figure 8C shows the specific cytotoxicity occurring *in vivo*, in various lymphoid and non-lymphoid organs, in mice immunized with DNA ("pSEM"), peptide ("ELA" = ELAGIGILTV) or an entrain/amplify sequence of DNA and peptide.

Figure 9A shows the persistence / decay of circulating tetramer stained T cells in animals immunized with peptide and amplified with peptide, along with the recall response following a peptide boost.

Figure 9B shows the persistence / decay of circulating tetramer stained T cells in animals entrained with DNA and amplified with peptide, along with the recall response following a peptide amplification.

Figure 9C shows the persistence / decay of circulating tetramer stained T cells in animals immunized with DNA and amplified with DNA, along with the recall response following a peptide boost.

Figure 10A shows the expansion of antigen-specific CD8+ T cells using various two-cycle immunization protocols.

Figure 10B shows the expansion of antigen-specific CD8+ T cells using various three-cycle immunization protocols.

Figure 10C shows the expansion of circulating antigen-specific T cells detected by tetramer staining, in animals primed using various protocols and amplified with peptide.

Figure 10D shows the expansion of antigen-specific T cells subsequent to various immunization regimens and detected by tetramer staining, in lymphoid and non-lymphoid organs.

Figure 11A shows an example of a schedule of immunizing mice with plasmid DNA and peptides

Figure 11B shows the immune response determined by ELISPOT analysis triggered by various immunization protocols (alternating DNA and peptide in respective or reverse order).

Figure 12A shows *in vivo* depletion of antigenic target cells, in blood and lymph nodes, in mice immunized with plasmid and peptide.

Figure 12B shows *in vivo* depletion of antigenic target cells, in spleen and lungs, in mice immunized with plasmid and peptide.

Figure 12C shows a summary of the results presented in 12A,B.

Figure 12D shows a correlation between frequency of specific T cells and *in vivo* clearance of antigenic target cells in mice immunized by the various protocols.

Figure 13A shows the schedule of immunizing mice with plasmid DNA and peptides, as well as the nature of measurements performed in those mice.

Figure 13B describes the schedule associated with the protocol used for determination of *in vivo* clearance of human tumor cells in immunized mice.

Figure 13C shows *in vivo* depletion of antigenic target cells (human tumor cells) in lungs of mice immunized with plasmid and peptide.

Figure 14A shows the immunization protocol used to generate the anti SSX-2 response shown in 14B.

Figure 14B shows the expansion of circulating SSX-2 specific T cells subsequent to applying a DNA entraining / peptide amplification regimen, detected by tetramer staining.

Figure 15A shows the *in vivo* clearance of antigenic target cells in spleens of mice that underwent various entrain-and-amplify protocols to simultaneously immunize against epitopes of melan A (ELAGIGILTV) and SSX2 (KASEKIFYV).

Figure 15B shows the *in vivo* clearance of antigenic target cells in the blood of mice that underwent various entrain-and-amplify protocols to simultaneously immunize against epitopes of melan A (ELAGIGILTV) and SSX2 (KASEKIFYV).

Figure 15C summarizes the results shown in detail in Figs 15A,B.

Figure 16 shows the expansion of the circulating antigen-specific CD8+ T cells measured by tetramer staining, in mice undergoing two cycles of various entrain-and-amplify protocols.

Figure 17A and B show the persistence of circulating antigen-specific T cells in animals undergoing two cycles of entrain-and-amplify protocols consisting of DNA/DNA/peptide (A) or DNA/peptide/peptide (B).

Figure 18 shows long-lived memory in animals undergoing two cycles of an entrain-and-amplify protocol consisting of DNA/DNA/DNA.

Figure 19 shows a clinical practice schema for enrollment and treatment of patients with DNA / peptide entrain-and-amplify protocols.

### Detailed Description of the Preferred Embodiment

This application claims priority under 35 U.S.C. § 119(e) to U.S. Provisional Application No. 60/479,393, filed on June 17, 2003, entitled METHODS TO CONTROL MHC CLASS I-RESTRICTED IMMUNE RESPONSE. The disclosure, including all methods, figures, and compositions, of 60/479,393 .

Embodiments of the present invention provide methods and compositions, for example, for generating immune cells specific to a target cell, for directing an effective immune response against a target cell, or for affecting/treating inflammatory disorders. The methods and compositions can include, for example, immunogenic compositions such as vaccines and therapeutics, and also prophylactic and therapeutic methods. Disclosed herein is the novel and unexpected discovery that by selecting the form of antigen, the sequence and timing with which it is administered, and delivering the antigen directly into secondary lymphoid organs, not only the magnitude, but the qualitative nature of the immune response can be managed.

Some preferred embodiments relate to compositions methods for entraining and amplifying a T cell response. For example such methods can include an entrainment step where a composition comprising a nucleic acid encoded immunogen is delivered to an animal. The composition can be delivered to various locations on the animal, but preferably is delivered to the lymphatic system, for example, a lymph node. The entrainment step can include one or more deliveries of the composition, for example, spread out over a period of time or in a continuous fashion over a period of time. Preferably, the methods can further include an amplification step comprising administering a composition comprising a peptide immunogen. The amplification step can be performed one or more times, for example, at intervals over a period of time, in one bolus, or continuously over a period of time. Although not required in all embodiments, some embodiments can include the use of compositions that include an immunopotentiator or adjuvant.

In some embodiments, depending on the nature of the immunogen and the context in which it is encountered, the immune response elicited can differ in its particular activity and makeup. In particular, while immunization with peptide can generate a cytotoxic/cytolytic T cell (CTL) response, attempts to further amplify this response with further injections can instead lead to the expansion of a regulatory T cell population, and a diminution of observable CTL activity. Thus compositions conferring high MHC/peptide concentrations on the cell surface within the lymph node, without additional immunopotentiating activity, can be used to purposefully promote a regulatory or tolerogenic response. In contrast immunogenic compositions providing ample immunopotentiation signals (*e.g*,. toll-like receptor ligands [or the cytokine/autocrine factors they would induce]) even if providing only limiting antigen, not only induce a response, but entrain it as well, so that subsequent encounters with ample antigen (*e.g*,. injected peptide) amplifies the response without changing the nature of the observed activity. Therefore, some embodiments relate to controlling the immune response profile, for example, the kind of response obtained and the kinds of cytokines produced. Some embodiments relate to methods and compositions for promoting the expansion or further expansion of CTL, and there are embodiment that relate to methods and compositions for promoting the expansion of regulatory cells in preference to the CTL, for example.

The disclosed methods are advantageous over many protocols that use only peptide or that do not follow the entrain-and-amplify methodology. As set forth above, many peptide-based immunization protocols and vector-based protocols have drawbacks. Nevertheless, if successful, a peptide based immunization or immune amplification strategy has advantages over other methods, particularly certain microbial vectors, for example. This is due to the fact that more complex vectors, such as live attenuated viral or bacterial vectors, may induce deleterious side-effects, for example, *in vivo* replication or recombination; or become ineffective upon repeated administration due to generation of neutralizing antibodies against the vector itself. Additionally, when harnessed in such a way to become strong immunogens, peptides can circumvent the need for proteasome-mediated processing (as with protein or more complex antigens, in context of "cross-processing" or subsequent to cellular infection). That is because cellular antigen processing for MHC-class I restricted presentation is a phenomenon that inherently selects dominant (favored) epitopes over subdominant epitopes, potentially interfering with the immunogenicity of epitopes corresponding to valid targets. Finally, effective peptide based immunization simplifies and shortens the process of development of immunotherapeutics.

Thus, effective peptide-based immune amplification methods, particularly including those described herein, can be of considerable benefit to immunotherapy (such as for cancer and chronic infections) or prophylactic vaccination (against certain infectious diseases). Additional benefits can be achieved by avoiding, particularly if simultaneous use of cumbersome, unsafe, or complex adjuvant techniques, although such techniques can be utilized in various embodiments described herein.

Definitions:

Unless otherwise clear from the context of the use of a term herein, the following listed terms shall generally have the indicated meanings for purposes of this description.

PROFESSIONAL ANTIGEN-PRESENTING CELL (pAPC) - a cell that possesses T cell costimulatory molecules and is able to induce a T cell response. Well characterized pAPCs include dendritic cells, B cells, and macrophages.

PERIPHERAL CELL - a cell that is not a pAPC.

HOUSEKEEPING PROTEASOME - a proteasome normally active in peripheral cells, and generally not present or not strongly active in pAPCs.

IMMUNOPROTEASOME - a proteasome normally active in pAPCs; the immunoproteasome is also active in some peripheral cells in infected tissues or following exposure to interferon.

EPITOPE - a molecule or substance capable of stimulating an immune response. In preferred embodiments, epitopes according to this definition include but are not necessarily limited to a polypeptide and a nucleic acid encoding a polypeptide, wherein the polypeptide is capable of stimulating an immune response. In other preferred embodiments, epitopes according to this definition include but are not necessarily limited to peptides presented on the surface of cells, the peptides being non-covalently bound to the binding cleft of class I MHC, such that they can interact with T cell receptors (TCR). Epitopes presented by class I MHC may be in immature or mature form. "Mature" refers to an MHC epitope in distinction to any precursor ("immature") that may include or consist essentially of a housekeeping epitope, but also includes other sequences in a primary translation product that are removed by processing, including without limitation, alone or in any combination, proteasomal digestion, N-terminal trimming, or the action of exogenous enzymatic activities. Thus, a mature epitope may be provided embedded in a somewhat longer polypeptide, the immunological potential of which is due, at least in part, to the embedded epitope; likewise, the mature epitope can be provided in its ultimate form that can bind in the MHC binding cleft to be recognized by TCR.

MHC EPITOPE - a polypeptide having a known or predicted binding affinity for a mammalian class I or class II major histocompatibility complex (MHC) molecule. Some particularly well characterized class I MHC molecules are presented in Tables 1-4.

HOUSEKEEPING EPITOPE - In a preferred embodiment, a housekeeping epitope is defined as a polypeptide fragment that is an MHC epitope, and that is displayed on a cell in which housekeeping proteasomes are predominantly active. In another preferred embodiment, a housekeeping epitope is defined as a polypeptide containing a housekeeping epitope according to the foregoing definition, that is flanked by one to several additional amino acids. In another preferred embodiment, a housekeeping epitope is defined as a nucleic acid that encodes a housekeeping epitope according to the foregoing definitions. Exemplary housekeeping epitopes are provided in U.S. Application Nos. 10/117,937, filed on April 4, 2002 (Pub. No. 20030220239 A1), and 10/657,022, and in PCT Application No. PCT/US2003/027706 (Pub. No. WO04022709A2), filed 9/5/2003; and U.S. Provisional Application Nos. 60/282,211, filed on April 6, 2001; 60/337,017, filed on November 7, 2001; 60/363210 filed 3/7/02; and 60/409,123, filed on September 5, 2002. Each of the listed applications is entitled EPITOPE SEQUENCES.

IMMUNE EPITOPE - In a preferred embodiment, an immune epitope is defined as a polypeptide fragment that is an MHC epitope, and that is displayed on a cell in which immunoproteasomes are predominantly active. In another preferred embodiment, an immune epitope is defined as a polypeptide containing an immune epitope according to the foregoing definition, that is flanked by one to several additional amino acids. In another preferred embodiment, an immune epitope is defined as a polypeptide including an epitope cluster sequence, having at least two polypeptide sequences having a known or predicted affinity for a class I MHC. In yet another preferred embodiment, an immune epitope is defined as a nucleic acid that encodes an immune epitope according to any of the foregoing definitions.

TARGET CELL - In a preferred embodiment, a target cells is a cell associated with a pathogenic condition that can be acted upon by the components of the immune system, for example, a cell infected with a virus or other intracellular parasite, or a neoplastic cell. In another embodiment, a target cell is a cell to be targeted by the vaccines and methods of the invention. Examples of target cells according to this definition include but are not necessarily limited to: a neoplastic cell and a cell harboring an intracellular parasite, such as, for example, a virus, a bacterium, or a protozoan. Target cells can also include cells that are targeted by CTL as a part of an assay to determine or confirm proper epitope liberation and processing by a cell expressing immunoproteasome, to determine T cell specificity or immunogenicity for a desired epitopes. Such cells can be transformed to express the liberation sequence, or the cells can simply be pulsed with peptide/epitope.

TARGET-ASSOCIATED ANTIGEN (TAA) - a protein or polypeptide present in a target cell.

TUMOR-ASSOCIATED ANTIGENS (TuAA) - a TAA, wherein the target cell is a neoplastic cell.

HLA EPITOPE - a polypeptide having a known or predicted binding affinity for a human class I or class II HLA complex molecule. Particularly well characterized class I HLAs are presented in Tables 1-4.

ANTIBODY - a natural immunoglobulin (Ig), poly- or monoclonal, or any molecule composed in whole or in part of an Ig binding domain, whether derived biochemically, or by use of recombinant DNA, or by any other means. Examples include *inter alia,* F(ab), single chain Fv, and Ig variable region-phage coat protein fusions.

SUBSTANTIAL SIMILARITY - this term is used to refer to sequences that differ from a reference sequence in an inconsequential way as judged by examination of the sequence. Nucleic acid sequences encoding the same amino acid sequence are substantially similar despite differences in degenerate positions or minor differences in length or composition of any non-coding regions. Amino acid sequences differing only by conservative substitution or minor length variations are substantially similar. Additionally, amino acid sequences comprising housekeeping epitopes that differ in the number of N-terminal flanking residues, or immune epitopes and epitope clusters that differ in the number of flanking residues at either terminus, are substantially similar. Nucleic acids that encode substantially similar amino acid sequences are themselves also substantially similar.

FUNCTIONAL SIMILARITY - this term is used to refer to sequences that differ from a reference sequence in an inconsequential way as judged by examination of a biological or biochemical property, although the sequences may not be substantially similar. For example, two nucleic acids can be useful as hybridization probes for the same sequence but encode differing amino acid sequences. Two peptides that induce cross-reactive CTL responses are functionally similar even if they differ by non-conservative amino acid substitutions (and thus may not be within the substantial similarity definition). Pairs of antibodies, or TCRs, that recognize the same epitope can be functionally similar to each other despite whatever structural differences exist. Testing for functional similarity of immunogenicity can be conducted by immunizing with the "altered" antigen and testing the ability of an elicited response, including but not limited to an antibody response, a CTL response, cytokine production, and the like, to recognize the target antigen. Accordingly, two sequences may be designed to differ in certain respects while retaining the same function. Such designed sequence variants of disclosed or claimed sequences are among the embodiments of the present invention.

EXPRESSION CASSETTE - a polynucleotide sequence encoding a polypeptide, operably linked to a promoter and other transcription and translation control elements, including but not limited to enhancers, termination codons, internal ribosome entry sites, and polyadenylation sites. The cassette can also include sequences that facilitate moving it from one host molecule to another.

EMBEDDED EPITOPE - in some embodiments, an embedded epitope is an epitope that is wholly contained within a longer polypeptide; in other embodiments, the term also can include an epitope in which only the N-terminus or the C-terminus is embedded such that the epitope is not wholly in an interior position with respect to the longer polypeptide.

MATURE EPITOPE - a peptide with no additional sequence beyond that present when the epitope is bound in the MHC peptide-binding cleft.

EPITOPE CLUSTER - a polypeptide, or a nucleic acid sequence encoding it, that is a segment of a protein sequence, including a native protein sequence, comprising two or more known or predicted epitopes with binding affinity for a shared MHC restriction element. In preferred embodiments, the density of epitopes within the cluster is greater than the density of all known or predicted epitopes with binding affinity for the shared MHC restriction element within the complete protein sequence. Epitope clusters are disclosed and more fully defined in U.S. Patent Application No. 09/561,571 entitled EPITOPE CLUSTERS.

LIBERATION SEQUENCE - a designed or engineered sequence comprising or encoding a housekeeping epitope embedded in a larger sequence that provides a context allowing the housekeeping epitope to be liberated by processing activities including, for example, immunoproteasome activity, N terminal trimming, and/or other processes or activities, alone or in any combination.

CTLp - CTL precursors are T cells that can be induced to exhibit cytolytic activity. Secondary *in vitro* lytic activity, by which CTLp are generally observed, can arise from any combination of naïve, effector, and memory CTL *in vivo.*

MEMORY T CELL - A T cell, regardless of its location in the body, that has been previously activated by antigen, but is in a quiescent physiologic state requiring re-exposure to antigen in order to gain effector function. Phenotypically they are generally CD62L⁻ CD44^{hi} CD107α⁻ IGN-γ⁻ LTβ⁻ TNF-α⁻ and is in GO of the cell cycle.

EFFECTOR T CELL - A T cell that, upon encountering antigen antigen, readily exhibits effector function. Effector T cells are generally capable of exiting the lymphatic system and entering the immunological periphery. Phenotypically they are generally CD62L⁻ CD44^{hi} CD107α⁺ IGN-γ⁺ LTβ⁺ TNF-α⁺ and actively cycling.

EFFECTOR FUNCTION - Generally, T cell activation generally, including acquisition of cytolytic activity and/or cytokine secretion.

INDUCING a T cell response - Includes in many embodiments the process of generating a T cell response from naïve, or in some contexts, quiescent cells; activating T cells.

AMPLIFYING a T cell response - Includes in many embodiments the process or increasing the number of cells, the number of activated cells, the level of activity, rate of proliferation, or similar parameter of T cells involved in a specific response.

ENTRAINMENT - Includes in many embodiments an induction that confers particular stability on the immune profile of the induced lineage of T cells.

TOLL-LIKE RECEPTOR (TLR) - Toll-like receptors (TLRs) are a family of pattern recognition receptors that are activated by specific components of microbes and certain host molecules. As part of the innate immune system, they contribute to the first line of defense against many pathogens, but also play a role in adaptive immunity.

TOLL-LIKE RECEPTOR (TLR) LIGAND - Any molecule capable of binding and activating a toll-like recepetor. Examples include, without limitation: poly IC A synthetic, double-stranded RNA know for inducing interferon. The polymer is made of one strand each of polyinosinic acid and polycytidylic acid, double-stranded RNA, unmethylated CpG oligodeoxyribonucleotide or other immunostimulatory sequences (ISSs), lipopolysacharide (LPS) , β-glucans, and imidazoquinolines, as well as derivatives and analogues thereof.

IMMUNOPOTENTIATING ADJCTVANTS - Adjuvants that activate pAPC or T cells including, for example: TLR ligands, endocytic-Pattern. Recognition Receptor (PRR) ligands, quillaja saponins, tucaresol, cytokines, and the like. Some preferred adjuvants are disclosed in Marciani, D.J. Drug Discovery Today 8:934-943, 2003.

IMMUNOSTIMULATORY SEQUENCE (ISS) - Generally an oligodeoxyribonucleotide containing an unmethlylated CpG sequence. The CpG may also be embedded in bacterially produced DNA, particularly plasmids. Further embodiments include various analogues; among preferred embodiments are molecules with one or more phosphorothioate bonds or non-physiologic bases.

VACCINE - In preferred embodiments a vaccine can be an immunogenic composition providing or aiding in prevention of disease. In other embodiments, a vaccine is a composition that can provide or aid in a cure of a disease. In others, a vaccine composition can provide or aid in amelioration of a disease. Further embodiments of a vaccine immunogenic composition can be used as therapeutic and/or prophylactic agents.

IMMUNIZATION - a process to induce partial or complete protection against a disease. Alternatively, a process to induce or amplify an immune system response to an antigen. In the second definition it can connote a protective immune response, particularly proinflammatory or active immunity, but can also include a regulatory response. Thus in some embodiments immunization is distinguished from tolerization (a process by which the immune system avoids producing proinflammatory or active immunity) while in other embodiments this term includes tolerization.

**Table 1**

| | Class I MHC Molecules |
|---|---|
| **Class I** | |
| **Human** | |
| HLA-A1 | |
| HLA-A*0101 | |
| HLA-A*0201 | |
| HLA-A*0202 | |
| HLA-A*0203 | |
| HLA-A*0204 | |
| HLA-A*0205 | |
| HLA-A*0206 | |
| HLA-A*0207 | |
| HLA-A*0209 | |
| HLA-A*0214 | |
| HLA-A3 | |
| HLA-A*0301 | |
| HLA-A*1101 | |
| HLA-A23 | |
| HLA-A24 | |
| HLA-A25 | |
| HLA-A*2902 | |
| HLA-A*3101 | |
| HLA-A*3302 | |
| HLA-A*6801 | |
| HLA-A*6901 | |
| HLA-B7 | |
| HLA-B*0702 | |
| HLA-B*0703 | |
| HLA-B*0704 | |
| HLA-B*0705 | |
| HLA-B8 | |
| HLA-B13 | |
| HLA-B14 | |
| HLA-B*1501 (B62) | |
| HLA-B17 | |
| HLA-B18 | |
| HLA-B22 | |
| HLA-B27 | |
| HLA-B*2702 | |
| HLA-B*2704 | |
| HLA-B*2705 | |
| HLA-B*2709 | |
| HLA-B35 | |
| HLA-B*3501 | |
| HLA-B*3502 | |
| HLA-B*3701 | |
| HLA-B*3801 | |
| HLA-B*39011 | |
| HLA-B*3902 | |
| HLA-B40 | |
| HLA-B*40012 (B60) | |
| HLA-B*4006 (B61) | |
| HLA-B44 | |
| HLA-B*4402 | |
| HLA-B*4403 | |
| HLA-B*4501 | |
| HLA-B*4601 | |
| HLA-B51 | |
| HLA-B*5101 | |
| HLA-B*5102 | |
| HLA-B*5103 | |
| HLA-B*5201 | |
| HLA-B*5301 | |
| HLA-B*5401 | |
| HLA-B*5501 | |
| HLA-B*5502 | |
| HLA-B*5601 | |
| HLA-B*5801 | |
| HLA-B*6701 | |
| HLA-B*7301 | |
| HLA-B*7801 | |
| HLA-Cw*0102 | |
| HLA-Cw*0301 | |
| HLA-Cw*0304 | |
| HLA-Cw*0401 | |
| HLA-Cw*0601 | |
| HLA-Cw*0602 | |
| HLA-Cw*0702 | |
| HLA-Cw8 | |
| HLA-Cw*1601 M | |
| HLA-G | |
| **Murine** | |
| H2-K^{d} | |
| H2-D^{d} | |
| H2-L^{d} | |
| H2-k^{b} | |
| H2-D^{b} | |
| H2-K^{k} | |
| H2-K^{kml} | |
| Qa-1^{a} | |
| Qa-2 | |
| H2-M3 | |
| **Rat** | |
| RT1.A^{a} | |
| RT1.A¹ | |
| **Bovine** | |
| Bota-A11 | |
| Bota-A20 | |
| **Chicken** | |
| B-F4 | |
| B-F12 | |
| B-F15 | |
| B-F19 | |
| **Chimpanzee** | |
| Patr-A*04 | |
| Patr-A*11 | |
| Patr-B*01 | |
| Patr-B*13 | |
| Patr-B*16 | |
| **Baboon** | |
| Papa-A*06 | |
| **Macaque** | |
| Mamu-A*01 | |
| **Swine** | |
| SLA (haplotype d/d) | |
| **Virus homolog** | |
| hCMV class I homolog UL18 | |

**Table 2**

| | Class I MHC Molecules |
|---|---|
| **Class I** | |
| **Human** | |
| HLA-A1 | |
| HLA-A*0101 | |
| HLA-A*0201 | |
| HLA-A*0202 | |
| HLA-A*0204 | |
| HLA-A*0205 | |
| HLA-A*0206 | |
| HLA-A*0207 | |
| HLA-A*0214 | |
| HLA-A3 | |
| HLA-A*1101 | |
| HLA-A24 | |
| HLA-A*2902 | |
| HLA-A*3101 | |
| HLA-A*3302 | |
| HLA-A*6801 | |
| HLA-A*6901 | |
| HLA-B7 | |
| HLA-B*0702 | |
| HLA-B*0703 | |
| HLA-B*0704 | |
| HLA-B*0705 | |
| HLA-B8 | |
| HLA-B14 | |
| HLA-B*1501 (B62) | |
| HLA-B27 | |
| HLA-B*2702 | |
| HLA-B*2705 | |
| HLA-B35 | |
| HLA-B*3501 | |
| HLA-B*3502 | |
| HLA-B*3701 | |
| HLA-B*3801 | |
| HLA-B*39011 | |
| HLA-B*3902 | |
| HLA-B40 | |
| HLA-B*40012 (B60) | |
| HLA-B*4006 (B61) | |
| HLA-B44 | |
| HLA-B*4402 | |
| HLA-B*4403 | |
| HLA-B*4601 | |
| HLA-B51 | |
| HLA-B*5101 | |
| HLA-B*5102 | |
| HLA-B*5103 | |
| HLA-B*5201 | |
| HLA-B*5301 | |
| HLA-B*5401 | |
| HLA-B*5501 | |
| HLA-B*5502 | |
| HLA-B*5601 | |
| HLA-B*5801 | |
| HLA-B*6701 | |
| HLA-B*7301 | |
| HLA-B*7801 | |
| HLA-Cw*0102 | |
| HLA-Cw*0301 | |
| HLA-Cw*0304 | |
| HLA-Cw*0401 | |
| HLA-Cw*0601 | |
| HLA-Cw*0602 | |
| HLA-Cw*0702 | |
| HLA-G | |
| **Murine** | |
| H2-K^{d} | |
| H2-D^{d} | |
| H2-L^{d} | |
| H2-K^{b} | |
| H2-D^{b} | |
| H2-K^{k} | |
| H2-K^{kml} | |
| Qa-2 | |
| **Rat** | |
| RT1.A^{a} | |
| RT1.A¹ | |
| **Bovine** | |
| Bota-A11 | |
| Bota-A20 | |
| **Chicken** | |
| B-F4 | |
| B-F12 | |
| B-F15 | |
| B-F19 | |
| **Virus homolog** | |
| hCMV class I homolog UL18 | |

**Table 3**

| Estimated gene frequencies of HLA-A antigens | | | | | | | | | | |
|---|---|---|---|---|---|---|---|---|---|---|
| Antigen | CAU | | AFR | | ASI | | LAT | | NAT | |
| | Gf^{a} | SE^{b} | Gf | SE | Gf | SE | Gf | SE | Gf | SE |
| A1 | 15.1843 | 0.0489 | 5.7256 | 0.0771 | 4.4818 | 0.0846 | 7.4007 | 0.0978 | 12.0316 | 0.2533 |
| A2 | 28.6535 | 0.0619 | 18.8849 | 0.1317 | 24.6352 | 0.1794 | 28.1198 | 0.1700 | 29.3408 | 0.3585 |
| A3 | 13.3890 | 0.0463 | 8.4406 | 0.0925 | 2.6454 | 0.0655 | 8.0789 | 0.1019 | 11.0293 | 0.2437 |
| A28 | 4.4652 | 0.0280 | 9.9269 | 0.0997 | 1.7657 | 0.0537 | 8.9446 | 0.1067 | 5.3856 | 0.1750 |
| A36 | 0.0221 | 0.0020 | 1.8836 | 0.0448 | 0.0148 | 0.0049 | 0.1584 | 0.0148 | 0.1545 | 0.0303 |
| A23 | 1.8287 | 0.0181 | 10.2086 | 0.1010 | 0.3256 | 0.0231 | 2.9269 | 0.0628 | 1.9903 | 0.1080 |
| A24 | 9.3251 | 0.0395 | 2.9668 | 0.0560 | 22.0391 | 0.1722 | 13.2610 | 0.1271 | 12.6613 | 0.2590 |
| A9 unsplit | 0.0809 | 0.0038 | 0.0367 | 0.0063 | 0.0858 | 0.0119 | 0.0537 | 0.0086 | 0.0356 | 0.0145 |
| A9 total | 11.2347 | 0.0429 | 13.2121 | 0.1128 | 22.4505 | 0.1.733 | 16.2416 | 0.1382 | 14.6872 | 0.2756 |
| A25 | 2.1157 | 0.0195 | 0.4329 | 0.0216 | 0.0990 | 0.0128 | 1.1937 | 0.0404 | 1.4520 | 0.0924 |
| A26 | 3.8795 | 0.0262 | 2.8284 | 0.0547 | 4.6628 | 0.0862 | 3.2612 | 0.0662 | 2.4292 | 0.1191 |
| A34 | 0.1508 | 0.0052 | 3.5228 | 0.0610 | 1.3529 | 0.0470 | 0.4928 | 0.0260 | 0.3150 | 0.0432 |
| A43 | 0.0018 | 0.0006 | 0.0334 | 0.0060 | 0.0231 | 0.0062 | 0.0055 | 0.0028 | 0.0059 | 0.0059 |
| A66 | 0.0173 | 0.0018 | 0.2233 | 0.0155 | 0.0478 | 0.0089 | 0.0399 | 0.0074 | 0.0534 | 0.0178 |
| A10 unsplit | 0.0790 | 0.0038 | 0.0939 | 0.0101 | 0.1255 | 0.0144 | 0.0647 | 0.0094 | 0.0298 | 0.0133 |
| A10 total | 6.2441 | 0.0328 | 7.1348 | 0.0850 | 6.3111 | 0.0993 | 5.0578 | 0.0816 | 4.2853 | 0.1565 |
| A29 | 3.5796 | 0.0252 | 3.2071 | 0.0582 | 1.1233 | 0.0429 | 4.5156 | 0.0774 | 3.4345 | 0.1410 |
| A30 | 2.5067 | 0.0212 | 13.0969 | 0.1129 | 2.2025 | 0.0598 | 4.4873 | 0.0772 | 2.5314 | 0.1215 |
| A31 | 2.7386 | 0.0221 | 1.6556 | 0.0420 | 3.6005 | 0.0761 | 4.8328 | 0.0800 | 6.0881 | 0.1855 |
| A32 | 3.6956 | 0.0256 | 1.5384 | 0.0405 | 1:0331 | 0.0411 | 2.7064 | 0.0604 | 2.5521 | 0.1220 |
| A33 | 1.2080 | 0.0148 | 6.5607 | 0.0822 | 9.2701 | 0.1191 | 2.6593 | 0.0599 | 1.0754 | 0.0796 |
| A74 | 0.0277 | 0.0022 | 1.9949 | 0.0461 | 0.0561 | 0.0096 | 0.2027 | 0.0167 | 0.1068 | 0.0252 |
| A19 unsplit | 0.0567 | 0.0032 | 0.2057 | 0.0149 | 0.0990 | 0.0128 | 0.1211 | 0.0129 | 0.0475 | 0.0168 |
| A19 total | 13.8129 | 0.0468 | 28.2593 | 0.1504 | 17.3846 | 0.1555 | 19.5252 | 0.1481 | 15.8358 | 0.2832 |
| AX | 0.8204 | 0.0297 | 4.9506 | 0.0963 | 2.9916 | 0.1177 | 1.6332 | 0.0878 | 1.8454 | 0.1925 |

| | | | | | | | | | | |
|---|---|---|---|---|---|---|---|---|---|---|
| ^{a}Gene frequency. ^{b}Standard error. | | | | | | | | | | |

**Table 4**

| Estimated gene frequencies for HLA-B antigens | | | | | | | | | | |
|---|---|---|---|---|---|---|---|---|---|---|
| Antigen | CAU | | AFR | | ASI | | LAT | | NAT | |
| | Gf^{a} | SE^{b} | Gf | SE | Gf | SE | Gf | SE | Gf | SE |
| B7 | 12.1782 | 0.0445 | 10.5960 | 0.1024 | 4.2691 | 0.0827 | 6.4477 | 0.0918 | 10.9845 | 0.2432 |
| B8 | 9.4077 | 0.0397 | 3.8315 | 0.0634 | 1.3322 | 0.0467 | 3.8225 | 0.0715 | 8.5789 | 0.2176 |
| B13 | 2.3061 | 0.0203 | 0.8103 | 0.0295 | 4.9222 | 0.0886 | 1.2699 | 0.0416 | 1.7495 | 0.1013 |
| B14 | 4.3481 | 0.0277 | 3.0331 | 0.0566 | 0.5004 | 0.0287 | 5.4166 | 0.0846 | 2.9823 | 0.1316 |
| B18 | 4.7980 | 0.0290 | 3.2057 | 0.0582 | 1.1246 | 0.0429 | 4.2349 | 0.0752 | 3.3422 | 0.1391 |
| B27 | 4.3831 | 0.0278 | 1.2918 | 0.0372 | 2.2355 | 0.0603 | 2.3724 | 0.0567 | 5.1970 | 0.1721 |
| B35 | 9.6614 | 0.0402 | 8.5172 | 0.0927 | 8.1203 | 0.1122 | 14.6516 | 0.1329 | 10.1198 | 0.2345 |
| B37 | 1.4032 | 0.0159 | 0.5916 | 0.0252 | 1.2327 | 0.0449 | 0.7807 | 0.0327 | 0.9755 | 0.0759 |
| B41 | 0.9211 | 0.0129 | 0.8183 | 0.0296 | 0.1303 | 0.0147 | 1.2818 | 0.0418 | 0.4766 | 0.0531 |
| B42 | 0.0608 | 0.0033 | 5.6991 | 0.0768 | 0.0841 | 0.0118 | 0.5866 | 0.0284 | 0.2856 | 0.0411 |
| B46 | 0.0099 | 0.0013 | 0.0151 | 0.0040 | 4.9292 | 0.0886 | 0.0234 | 0.0057 | 0.0238 | 0.0119 |
| B47 | 0.2069 | 0.0061 | 0.1305 | 0.0119 | 0.0956 | 0.0126 | 0.1832 | 0.0159 | 0.2139 | 0.0356 |
| B48 | 0.0865 | 0.0040 | 0.1316 | 0.0119 | 2.0276 | 0.0575 | 1.5915 | 0.0466 | 1.0267 | 0.0778 |
| B53 | 0.4620 | 0.0092 | 10.9529 | 0.1039 | 0.4315 | 0.0266 | 1.6982 | 0.0481 | 1.0804 | 0.0798 |
| B59 | 0.0020 | 0.0006 | 0.0032 | 0.0019 | 0.4277 | 0.0265 | 0.0055 | 0.0028 | 0^{c} | |
| B67 | 0.0040 | 0.0009 | 0.0086 | 0.0030 | 0.2276 | 0.0194 | 0.0055 | 0.0028 | 0.0059 | 0.0059 |
| B70 | 0.3270 | 0.0077 | 7.3571 | 0.0866 | 0.8901 | 0.0382 | 1.9266 | 0.0512 | 0.6901 | 0.0639 |
| B73 | 0.0108 | 0.0014 | 0.0032 | 0.0019 | 0.0132 | 0.0047 | 0.0261 | 0.0060 | 0^{c} | |
| B51 | 5.4215 | 0.0307 | 2.5980 | 0.0525 | 7.4751 | 0.1080 | 6.8147 | 0.0943 | 6.9077 | 0.1968 |
| B52 | 0.9658 | 0.0132 | 1.3712 | 0.0383 | 3.5121 | 0.0752 | 2.2447 | 0.0552 | 0.6960 | 0.0641 |
| B5 unsplit | 0.1565 | 0.0053 | 0.1522 | 0.0128 | 0.1288 | 0.0146 | 0.1546 | 0.0146 | 0.1307 | 0.0278 |
| B5 total | 6.5438 | 0.0435 | 4.1214 | 0.0747 | 11.1160 | 0.1504 | 9.2141 | 0.1324 | 7.7344 | 0.2784 |
| B44 | 13.4838 | 0.0465 | 7.0137 | 0.0847 | 5.6807 | 0.0948 | 9.9253 | 0.1121 | 11.8024 | 0.2511 |
| B45 | 0.5771 | 0.0102 | 4.8069 | 0.0708 | 0.1816 | 0.0173 | 1.8812 | 0.0506 | 0.7603 | 0.0670 |
| B12 unsplit | 0.0788 | 0.0038 | 0.0280 | 0.0055 | 0.0049 | 0.0029 | 0.0193 | 0.0051 | 0.0654 | 0.0197 |
| B12 total | 14.1440 | 0.0474 | 11.8486 | 0.1072 | 5.8673 | 0.0963 | 11.8258 | 0.1210 | 12.6281 | 0.2584 |
| B62 | 5.9117 | 0.0320 | 1.5267 | 0.0404 | 9.2249 | 0.1190 | 4.1825 | 0.0747 | 6.9421 | 0.1973 |
| B63 | 0.4302 | 0.0088 | 1.8865 | 0.0448 | 0.4438 | 0.0270 | 0.8083 | 0.0333 | 0.3738 | 0.0471 |
| B75 | 0.0104 | 0.0014 | 0.0226 | 0.0049 | 1.9673 | 0.0566 | 0.1101 | 0.0123 | 0.0356 | 0.0145 |
| B76 | 0.0026 | 0.0007 | 0.0065 | 0.0026 | 0.0874 | 0.0120 | 0.0055 | 0.0028 | 0 | |
| B77 | 0.0057 | 0.0010 | 0.0119 | 0.0036 | 0.0577 | 0.0098 | 0.0083 | 0.0034 | 0^{c} | 0.0059 |
| B15 unsplit | 0.1305 | 0.0049 | 0.0691 | 0.0086 | 0.4301 | 0.0266 | 0.1820 | 0.0158 | 0.0059 | 0.0206 |
| B15 total | 6.4910 | 0.0334 | 3.5232 | 0.0608 | 12.2112 | 0.1344 | 5.2967 | 0.0835 | 0.0715 7.4290 | 0.2035 |
| B38 | 2.4413 | 0.0209 | 0.3323 | 0.0189 | 3.2818 | 0.0728 | 1.9652 | 0.0517 | 1.1017 | 0.0806 |
| B39 | 1.9614 | 0.0188 | 1.2893 | 0.0371 | 2.0352 | 0.0576 | 6.3040 | 0.0909 | 4.5527 | 0.1615 |
| B16 unsplit | 0.0638 | 0.0034 | 0.0237 | 0.0051 | 0.0644 | 0.0103 | 0.1226 | 0.0130 | 0.0593 | 0.0188 |
| B16 total | 4.4667 | 0.0280 | 1.6453 | 0.0419 | 5.3814 | 0.0921 | 8.3917 | 0.1036 | 5.7137 | 0.1797 |
| B57 | 3.5955 | 0.0252 | 5.6746 | 0.0766 | 2.5782 | 0.0647 | 2.1800 | 0.0544 | 2.7265 | 0.1260 |
| B58 | 0.7152 | 0.0114 | 5.9546 | 0.0784 | 4.0189 | 0.0803 | 1.2481 | 0.0413 | 0.9398 | 0.0745 |
| B 17 unsplit | 0.2845 | 0.0072 | 0.3248 | 0.0187 | 0.3751 | 0.0248 | 0.1446 | 0.0141 | 0.2674 | 0.0398 |
| B17 total | 4.5952 | 0.0284 | 11.9540 | 0.1076 | 6.9722 | 0.1041 | 3.5727 | 0.0691 | 3.9338 | 0.1503 |
| B49 | 1.6452 | 0.0172 | 2.6286 | 0.0528 | 0.2440 | 0.0200 | 2.3353 | 0.0562 | 1.5462 | 0.0953 |
| B50 | 1.0580 | 0.0138 | 0.8636 | 0.0304 | 0.4421 | 0.0270 | 1.8883 | 0.0507 | 0.7862 | 0.0681 |
| B21 unsplit | 0.0702 | 0.0036 | 0.0270 | 0.0054 | 0.0132 | 0.0047 | 0.0771 | 0.0103 | 0.0356 | 0.0145 |
| B21 total | 2.7733 | 0.0222 | 3.5192 | 0.0608 | 0.6993 | 0.0339 | 4.3007 | 0.0755 | 2.3680 | 0.1174 |
| B54 | 0.0124 | 0.0015 | 0.0183 | 0.0044 | 2.6873 | 0.0660 | 0.0289 | 0.0063 | 0.0534 | 0.0178 |
| B55 | 1.9046 | 0.0185 | 0.4895 | 0.0229 | 2.2444 | 0.0604 | 0.9515 | 0.0361 | 1.4054 | 0.0909 |
| B56 | 0.5527 | 0.0100 | 0.2686 | 0.0170 | 0.8260 | 0.0368 | 0.3596 | 0.0222 | 0.3387 | 0.0448 |
| B22 unsplit | 0.1682 | 0.0055 | 0.0496 | 0.0073 | 0.2730 | 0.0212 | 0.0372 | 0.0071 | 0.1246 | 0.0272 |
| B22 total | 2.0852 | 0.0217 | 0.8261 | 0.0297 | 6.0307 | 0.0971 | 1.3771 | 0.0433 | 1.9221 | 0.1060 |
| B60 | 5.2222 | 0.0302 | 1.5299 | 0.0404 | 8.3254 | 0.1135 | 2.2538 | 0.0553 | 5.7218 | 0.1801 |

| Antigen | CAU | | AFR | | ASI | | LAT | | NAT | |
|---|---|---|---|---|---|---|---|---|---|---|
| | Gf^{a} | SE^{b} | Gf | SE | Gf | SE | Gf | SE | Gf | SE |
| B61 | 1.1916 | 0.0147 | 0.4709 | 0.0225 | 6.2072 | 0.0989 | 4.6691 | 0.0788 | 2.6023 | 0.1231 |
| B40 unsplit | 0.2696 | 0.0070 | 0.0388 | 0.0065 | 0.3205 | 0.0230 | 0.2473 | 0.0184 | 0.2271 | 0.0367 |
| B40 total | 6.6834 | 0.0338 | 2.0396 | 0.0465 | 14.8531 | 0.1462 | 7.1702 | 0.0963 | 8.5512 | 0.2168 |
| BX | 1.0922 | 0.0252 | 3.5258 | 0.0802 | 3.8749 | 0.0988 | 2.5266 | 0.0807 | 1.9867 | 0.1634 |

| | | | | | | | | | | |
|---|---|---|---|---|---|---|---|---|---|---|
| ^{a}Gene frequency. ^{b}Standard error. ^{c}The observed gene count was zero. | | | | | | | | | | |

**Table 5**

| Listing of CT genes*: | | |
|---|---|---|
| **CT Identifier** | **Transcript / Transcript family** | **Family Members/CT Identifier (Synonyms)** |
| CT1 | MAGEA | MAGEA1/CT1.1, MAGEA2/CT1.2, MAGEA3/CT1.3, MAGEA4/CT1.4, MAGEA5/CT1.5, MAGEA6/CT1.6, MAGEA7/CT1.7, MAGEAB/CT1.8, MAGEA9/CT.9, MAGEA10/CT1.10, MAGEA11/CT1.11, MAGEA12/CT1.12 |
| CT2 | BAGE | BAGE/CT2.1, BAGE2/CT2.2, BAGE3/CT2.3, BAGE4/CT2.4, BAGE5/CT2.5 |
| CT3 | MAGEB | MAGEB1/CT3.1, MAGEB2/CT3.2, MAGEB5/CT3.3, MAGEB6/CT3.4 |
| CT4 | GAGE1 | GAGE1/CT4.1, GAGE2/CT4.2, GAGE3/CT4.3, GAGE4/CT4.4, GAGE5/CT4.5, GAGE6/CT4.6, GAGE7/CT4.7, GAGE8/CT4.8 |
| CT5 | SSX | SSX1/CT5.1, SSX2/CT5.2a, SSX2/CT5.2b, SSX3/CT5.3, SSX4/CT5.4 |
| CT6 | NY-ESO-1 | NY-ESO-1/CT6.1, LAGE-1a/CT6.2a, LAGE-lb/CT6.2b |
| CT7 | MAGEC1 | MAGEC1/CT7.1, MAGEC3/CT7.2 |
| CT8 | SYCP1 | SYCP1/CT8 |
| CT9 | BRDT | BRDT/CT9 |
| CT10 | MAGEE1 | MAGEE1/CT10 |
| CT11 | CUP11/SPANX | SPANXA1/CT11.1, SPANXB1/CT11.2, SPANXC/CT11.3, SPANXD/CT11.4 |
| CT12 | XAGE-1/GAGED | XAGE-1a/CT12.1a, XAGE-1b/CT12.1b, XAGE-1c/CT12.1c, XAGE-1d/CT12.1d, XAGE-2/CT12.2, XAGE-3a/CT12.3a, XAGE-3b/CT12.3b, XAGE-4/CT12.4 |
| CT13 | HAGE | HAGE/CT13 |
| CT14 | SAGE | SAGE/CT14 |
| CT15 | ADAM2 | ADAM2/CT15 |
| CT16 | PAGE-5 | PAGE-5/CT16.1, CT16.2 |
| CT17 | LIP 1 | LIP1/CT17 |
| CT18 | NA88 | NA88/CT12 |
| CT19 | IL13RA1 | IL13RA1/CT19 |
| CT20 | TSP50 | TSP50/CT20 |
| CT21 | CTAGE-1 | CTAGE-1/CT21.1, CTAGE-2/CT21.2 |
| CT22 | SPA17 | SPA17/CT22 |
| CT23 | OY-TES-1 | OY-TES-1/CT23 |
| CT24 | CSAGE | CSAGE/CT24.1, TRAG3/CT24.2 |
| CT25 | MMA1/DSCR8 | MMA-1a/CT25.1a, MMA-1b/CT25.1b |
| CT26 | CAGE | CAGE/CT26 |
| CT27 | BORIS | BORIS/CT27 |
| CT28 | HOM-TES-85 | HOM-TES-85/CT28 |
| CT29 | AF15q14/D40 AF15q14/D40 | D40/CT29 |
| CT30 | E2F-like/HCA661 | HCA661/CT30 |
| CT31 | PLU-1 | PLU-1/CT31 |
| CT32 | LDHC | LDHC/CT32 |
| CT33 | MORC | MORC/CT33 |
| CT34 | SGY-1 | SGY-1/CT34 |
| CT35 | SPO11 | SPO11/CT35 |
| CT36 | TPX1 | TPX-1/CT36 |
| CT37 | NY-SAR-35 | NY-SAR-35/CT37 |
| CT38 | FTHL17 | FTHL17/CT38 |
| CT39 | NXF2 | NXF2/CT39 |
| CT40 | TAF7L | TAF7L/CT40 |
| CT41 | TDRD1 | TDRD1/CT41.1, NY-CO-45/CT41.2 |
| CT42 | TEX15 | TEX15/CT42 |
| CT43 | FATE | FATE/CT43 |
| CT44 | TPTE | TPTE/CT44 |
| --- | PRAME | (MAPE, DAGE) |

| | | |
|---|---|---|
| **See* Scanlan et al., "The cancer/testis genes: Review, standardization, and commentary," Cancer Immunity, Vol. 4, p. 1 (23 January 2004). | | |

The following discussion sets forth the inventors' understanding of the operation of the invention. However, it is not intended that this discussion limit the patent to any particular theory of operation not set forth in the claims.

Effective immune-mediated control of tumoral processes or microbial infections generally involves induction and expansion of antigen-specific T cells endowed with multiple capabilities such as migration, effector functions, and differentiation into memory cells. Induction of immune responses can be attempted by various methods and involves administration of antigens in different forms, with variable effect on the magnitude and quality of the immune response. One limiting factor in achieving a control of the immune response is targeting pAPC able to process and effectively present the resulting epitopes to specific T cells.

A solution to this problem is direct antigen delivery to secondary lymphoid organs, a microenvironment abundant in pAPC and T cells. The antigen can be delivered, for example, either as polypeptide or as an expressed antigen by any of a variety of vectors) The outcome in terms of magnitude and quality of immunity can be controlled by factors including, for example, the dosage, the formulation, the nature of the vector, and the molecular environment. Embodiments of the present invention can enhance control of the immune response. Control of the immune response includes the capability to induce different types of immune responses as needed, for example, from regulatory to pro-inflammatory responses. Preferred embodiments provide enhanced control of the magnitude and quality of responses to MHC class I-restricted epitopes which are of major interest for active immunotherapy.

Previous immunization methods displayed certain important limitations: first, very often, conclusions regarding the potency of vaccines were extrapolated from immunogenicity data generated from one or from a very limited panel of ultrasensitive read-out assays. Frequently, despite the inferred potency of a vaccination regimen, the clinical response was not significant or was at best modest. Secondly, subsequent to immunization, T regulatory cells, along with more conventional T effector cells, can be generated and/or expanded, and such cells can interfere with the function of the desired immune response. The importance of such mechanisms in active immunotherapy has been recognized only recently.

Intranodal administration of immunogens provides a basis for the control of the magnitude and profile of immune responses. The effective *in vivo* loading of pAPC accomplished as a result of such administration, enables a substantial magnitude of immunity, even by using an antigen in its most simple form-a peptide epitope-otherwise generally associated with poor pharmocokinetics. The quality of response can be further controlled via the nature of immunogens, vectors, and protocols of immunization. Such protocols can be applied for enhancing/modifying the response in chronic infections or tumoral processes.

Immunization has traditionally relied on repeated administration of antigen to augment the magnitude of the immune response. The use of DNA vaccines has resulted in high quality responses, but it has been difficult to obtain high magnitude responses using such vaccines, even with repeated booster doses. Both characteristics of the response, high quality and low magnitude, are likely due to the relatively low levels of epitope loading onto MHC achieved with these vectors. Instead it has become more common to boost such vaccines using antigen encoded in a live virus vector in order to achieve the high magnitude of response needed for clinical usefulness. However, the use of live vectors can entail several drawbacks including potential safety issues, decreasing effectiveness of later boosts due to a humoral response to the vector induced by the prior administrations, and the costs of creation and production. Thus, use of live vectors or DNA alone, although eliciting high quality responses, may result in a limited magnitude or sustainability of response.

Disclosed herein are embodiments that relate to protocols and to methods that, when applied to peptides, rendered them effective as immune therapeutic tools. Such methods circumvent the poor PK of peptides, and if applied in context of specific, and often more complex regimens, result in robust amplification and/or control of immune response. In preferred embodiments, direct administration of peptide into lymphoid organs results in unexpectedly strong amplification of immune responses, following a priming agent that induces a strong, moderate or even mild (at or below levels of detection by conventional techniques) immune response consisting of Tc1 cells. While preferred embodiments of the invention can employ intralymphatic administration of antigen at all stages of immunization, intralymphatic administration of adjuvant-free peptide is most preferred. Peptide amplification utilizing intralymphatic administration can be applied to existing immune responses that may have been previously induced. Previous induction can occur by means of natural exposure to the antigen or by means of commonly used routes of administration, including without limitation subcutaneous, intradermal, intraperitoneal, intramuscular, and mucosal.

Also as shown herein, optimal initiation, resulting in subsequent expansion of specific T cells, can be better achieved by exposing the naive T cells to limited amounts of antigen (as can result from the often limited expression of plasmid-encoded antigen) in a rich co-stimulatory context (such as in a lymph node). That can result in activation of T cells carrying T cell receptors that recognize with high affinity the MHC - peptide complexes on antigen presenting cells and can result in generation of memory cells that are more reactive to subsequent stimulation. The beneficial co-stimulatory environment can be augmented or ensured through the use of immunopotentiating agents and thus intralymphatic administration, while advantageous, is not in all embodiments required for initiation of the immune response.

While the poor pharmacokinetics of free peptides has prevented their use in most routes of administration, direct administration into secondary lymphoid organs, particularly lymph nodes, has proven effective when the level of antigen is maintained more or less continuously by continuous infusion or frequent (for example, daily) injection. Such intranodal administration for the generation of CTL is taught in U.S. Patent applications 09/380,534 and 09/776,232 (Pub. No. 20020007173 A1), and in PCT Application No. PCTUS98/14289 (Pub. No. WO9902183A2) each entitled A METHOD OF INDUCING A CTL RESPONSE. In some embodiments of the instant invention, intranodal administration of peptide was effective in amplifying a response initially induced with a plasmid DNA vaccine. Moreover, the cytokine profile was distinct, with plasmid DNA induction/peptide amplification generally resulting in greater chemokine (chemoattractant cytokine) and lesser immunosuppressive cytokine production than either DNA/DNA or peptide/peptide protocols.

Thus, such DNA induction/peptide amplification protocols can improve the effectiveness of compositions, including therapeutic vaccines for cancer and chronic infections. Beneficial epitope selection principles for such immunotherapeutics are disclosed in U.S. patent application Nos. 09/560,465, 10/026,066 (Pub. No. 20030215425 A1), and 10/005,905 all entitled EPITOPE SYNCHRONIZATION IN ANTIGEN PRESENTING CELLS; 09/561,074 entitled METHOD OF EPITOPE DISCOVERY; 09/561,571 entitled EPITOPE CLUSTERS; 10/094,699 (Pub. No. 20030046714 A1) entitled ANTI-NEOVASCULATURE PREPARATIONS FOR CANCER; and 10/117,937 (Pub. No. 20030220239 A1) and 10/657,022, and PCT Application No. PCT/US2003/027706 (Pub. No. WO04022709A2) both entitled EPITOPE SEQUENCES. Aspects of the overall design of vaccine plasmids are disclosed in U.S. Patent applications 09/561,572 entitled EXPRESSION VECTORS ENCODING EPITOPES OF TARGET-ASSOCIATED ANTIGENS and 10/292,413 (Pub. No.20030228634 A1) entitled EXPRESSION VECTORS ENCODING EPITOPES OF TARGET-ASSOCIATED ANTIGENS AND METHODS FOR THEIR DESIGN; 10/225,568 (Pub No. 2003-0138808), PCT Application No. PCT/US2003/026231 (Pub. No. WO 2004/018666) and U.S. Patent No. 6,709,844, entitled AVOIDANCE OF UNDESIRABLE REPLICATION INTERMEDIATES IN PLASMIND PROPAGATION. Specific antigenic combinations of particular benefit in directing an immune response against particular cancers are disclosed in provisional U.S. patent application No. 60/479,554 and U.S. patent application No. / (Attorney Docket No: MANNK.035A) and PCT Patent Application No. (Pub. No. ), both entitled COMBINATIONS OF TUMOR-ASSOCIATED ANTIGENS IN VACCINES FOR VARIOUS TYPES OF CANCERS filed on June 17, 2003 and on even date with this application, respectively.

Surprisingly, repeated intranodal injection of peptide according to a traditional prime-boost schedule resulted in reducing the magnitude of the cytolytic response compared to response observed after initial dosing alone. Examination of the immune response profile shows this to be the result of the induction of immune regulation (suppression) rather than unresponsiveness. This is in contrast to induce-and-amplify protocols encompassing DNA-encoded immunogens, typically plasmids. Direct loading of pAPC by intranodal injection of antigen generally diminishes or obviates the need for adjuvants that are commonly used to correct the pharmacokinetics of antigens delivered via other parenteral routes. The absence of such adjuvants, which are generally proinflammatory, can thus facilitate the induction of a different (i.e., regulatory or tolerogenic) immune response profile than has previously been observed with peptide immunization. Since the response, as shown in the examples below, is measured in secondary lymphoid organs remote from the initial injection site, such results support the use methods and compositions according to of the embodiments of the invention for modifying (suppressing) ongoing inflammatory reactions. This approach can be useful even with inflammatory disorders that have a class II MHC-restricted etiology, either by targeting the same antigen, or any suitable antigen associated with the site of inflammation, and relying on bystander effects mediated by the immunosuppressive cytokines.

Despite the fact that repeated peptide administration results in gradually decreasing cytolytic immune response, induction with an agent such as non-replicating recombinant DNA (plasmid) had a substantial impact on the subsequent doses, enabling robust amplification of immunity to epitopes expressed by the recombinant DNA and peptide, and entraining its cytolytic nature. In fact, when single or multiple administrations of recombinant DNA vector or peptide separately achieved no or modest immune responses, inducing with DNA and amplifying with peptide achieved substantially higher responses, both as a rate of responders and as a magnitude of response. In the examples shown, the rate of response was at least doubled and the magnitude of response (mean and median) was at least tripled by using a recombinant DNA induction / peptide -amplification protocol. Thus, preferred protocols result in induction of immunity (Tc1 immunity) that is able to deal with antigenic cells *in vivo,* within lymphoid and non-lymphoid organs. One limiting factor in most cancer immunotherapy is the limited susceptibility of tumor cells to immune-mediated attack, possibly due to reduced MHC/peptide presentation. In preferred embodiments, robust expansion of immunity is achieved by DNA induction / peptide amplification, with a magnitude that generally equals or exceeds the immune response generally observed subsequent to infection with virulent microbes. This elevated magnitude can help to compensate for poor MHC/peptide presentation and does result in clearance of human tumor cells as shown in specialized pre-clinical models such as, for example, HLA transgenic mice.

Such induce-and-amplify protocols involving specific sequences of recombinant DNA entrainment doses, followed by peptide boosts administered to lymphoid organs, are thus useful for the purpose of induction, amplification and maintenance of strong T cell responses, for example for prophylaxis or therapy of infectious or neoplastic diseases. Such diseases can be carcinomas (*e.g*., renal, ovarian, breast, lung, colorectal, prostate, head-and-neck, bladder, uterine, skin), melanoma, tumors of various origin and in general tumors that express defined or definable tumor associated antigens, such as oncofetal (*e.g*., CEA, CA 19-9, CA 125, CRD-BP, Das-1, 5T4, TAG-72, and the like), tissue differentiation (*e.g*.,melan-A, tyrosinase, gp100, PSA, PSMA, and the like), or cancer-testis antigens (*e.g*.,PRAME, MAGE, LAGE, SSX2, NY-ESO-1, and the like; see Table 5). Cancer-testis genes and their relevance for cancer treatment are reviewed in Scanlon et al., Cancer Immunity 4:1-15, 2004), Antigens associated with tumor neovasculature (*e.g*.,PSMA, VEGFR2, Tie-2) are also useful in connection with cancerous diseases, as is disclosed in U.S. Patent Application No. 10/094,699 (Pub. No. 20030046714 A1), entitled ANTI-NEOVASCULATURE PREPARATIONS FOR CANCER. The methods and compositions can be used to target various organisms and disease conditions. For example, the target organisms can include bacteria, viruses, protozoa, fungi, and the like. Target diseases can include those caused by prions, for example. Exemplary diseases, organisms and antigens and epitopes associated with target organisms, cells and diseases are described in U.S. Application No. 09/776,232 (Pub. No. 20020007173 A1)Among the infectious diseases that can be addressed are those caused by agents that tend to establish chronic infections (HIV, herpes simplex virus, CMV, Hepatitis B and C viruses, papilloma virus and the like) and/or those that are connected with acute infections (for example, influenza virus, measles, RSV, Ebola virus). Of interest are viruses that have oncogenic potential - from the perspective of prophylaxis or therapy - such as papilloma virus, Epstein Barr virus and HTLV-1. All these infectious agents have defined or definable antigens that can be used as basis for designing compositions such as peptide epitopes.

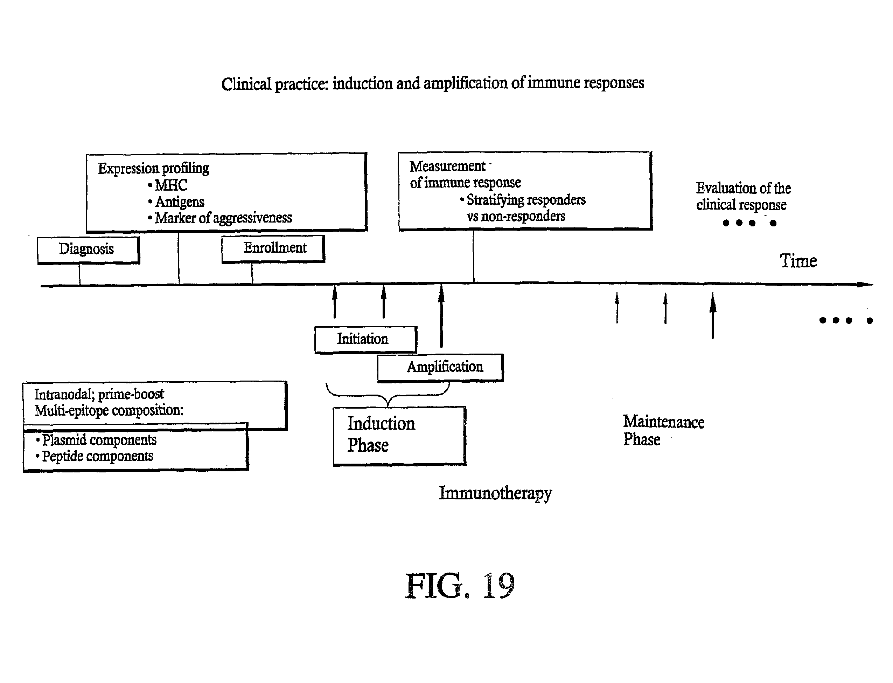

Preferred applications of such methods (*See, e.g.,* Figure 19) include injection or infusion into one or more lymph nodes, starting with a number (*e.g*., 1 to 10, or more, 2 to 8, 3 to 6, preferred about 4 or 5) of administrations of recombinant DNA (dose range of 0.001 - 10 mg/kg, preferred 0.005-5mg/kg) followed by one or more (preferred about 2) administrations of peptide, preferably in an immunologically inert vehicle or formulation (dose range of 1 ng/kg - 10 mg/kg, preferred 0.005-5 mg/kg). Because dose does not necessarily scale linearly with the size of the subject, doses for humans can tend toward the lower, and doses for mice can tend toward the higher, portions of these ranges. The preferred concentration of plasmid and peptide upon injection is generally about 0.1µg/ml-10 mg/ml, and the most preferred concentration is about 1mg/ml, generally irrespective of the size or species of the subject. However, particularly potent peptides can have optimum concentrations toward the low end of this range, for example between 1 and 100 µg/ml. When peptide only protocols are used to promote tolerance doses toward the higher end of these ranges are generally preferred (*e.g*., 0.5-10 mg/ml). This sequence can be repeated as long as necessary to maintain a strong immune response *in vivo.* Moreover, the time between the last entraining dose of DNA and the first amplifying dose of peptide is not critical. Preferably it is about 7 days or more, and can exceed several months. The multiplicity of injections of the DNA and/or the peptide can be reduced by substituting infusions lasting several days (preferred 2-7 days). It can be advantageous to initiate the infusion with a bolus of material similar to what might be given as an injection, followed by a slow infusion (24-12000 µl/day to deliver about 25-2500 µg/day for DNA, 0.1 - 10,000 µg/day for peptide). This can be accomplished manually or through the use of a programmable pump, such as an insulin pump. Such pumps are known in the art and enable periodic spikes and other dosage profiles, which can be desirable in some embodiments.

It should be noted that while this method successfully makes use of peptide, without conjugation to proteins, addition of adjuvant, etc., in the amplification step, the absence of such components is not required. Thus, conjugated peptide, adjuvants, immunopentiators, etc. can be used in embodiments. More complex compositions of peptide administered to the lymph node, or with an ability to home to the lymphatic system, including peptide-pulsed dendritic cells, suspensions such as liposome formulations, aggregates, emulsions, microparticles, nanocrystals, composed of or encompassing peptide epitopes or antigen in various forms, can be substituted for free peptide in the method. Conversely, peptide boost by intranodal administration can follow priming via any means / or route that achieves induction of T memory cells even at modest levels.

In order to reduce occurrence of resistance due to mosaicism of antigen expression, or to mutation or loss of the antigen, it is advantageous to immunize to multiple, preferably about 2-4, antigens concomitantly. Any combination of antigens can be used. A profile of the antigen expression of a particular tumor can be used to determine which antigen or combination of antigens to use. Exemplary methodology is found in U.S. Provisional Application No. / , (Attorney Docket: MANNK.035PR2), filed on even date herewith, entitled "COMBINATIONS OF TUMOR-ASSOCIATED ANTIGENS IN DIAGNOTISTICS FOR VARIOUS TYPES OF CANCERS;". Specific combinations of antigens particularly suitable to treatment of selected cancers are disclosed in U.S. patent applications 60/479,554 and / (Atty. Docket No. MANNK.035A) and PCT Application No. (Pub. No. ), cited and incorporated by reference above. To trigger immune responses to a plurality of antigens or to epitopes from a single antigen, these methods can be used to deliver multiple immunogenic entities, either individually or as mixtures. When immunogens are delivered individually, it is preferred that the different entities be administered to different lymph nodes or to the same lymph node(s) at different times, or to the same lymph node(s) at the same time. This can be particularly relevant to the delivery of peptides for which a single formulation providing solubility and stability to all component peptides can be difficult to devise. A single nucleic acid molecule can encode multiple immunogens. Alternatively, multiple nucleic acid molecules encoding one or a subset of all the component immunogens for the plurality of antigens can be mixed together so long as the desired dose can be provided without necessitating such a high concentration of nucleic acid that viscosity becomes problematic.

In preferred embodiments the method calls for direct administration to the lymphatic system. In preferred embodiments this is to a lymph node. Afferent lymph vessels are similarly preferred. Choice of lymph node is not critical. Inguinal lymph nodes are preferred for their size and accessibility, but axillary and cervical nodes and tonsils can be similarly advantageous. Administration to a single lymph node can be sufficient to induce or amplify an immune response. Administration to multiple nodes can increase the reliability and magnitude of the response.

Patients that can benefit from such methods of immunization can be recruited using methods to define their MHC protein expression profile and general level of immune responsiveness. In addition, their level of immunity can be monitored using standard techniques in conjunction with access to peripheral blood. Finally, treatment protocols can be adjusted based on the responsiveness to induction or amplification phases and variation in antigen expression. For example, repeated entrainment doses preferably can be administered until a detectable response is obtained, and then administering the amplifying peptide dose(s), rather than amplifying after some set number of entrainment doses. Similarly, scheduled amplifying or maintenance doses of peptide can be discontinued if their effectiveness wanes, antigen-specific regulatory T cell numbers rise, or some other evidence of tolerization is observed, and further entrainment can be administered before resuming amplification with the peptide. The integration of diagnostic techniques to assess and monitor immune responsiveness with methods of immunization is discussed more fully in Provisional U.S. Patent Application No. / (Atty. Docket No. MANNK.040PR), entitled IMPROVED EFFICACY OF ACTIVE IMMUNOTHERAPY BY INTEGRATING DIAGNOSTIC WITH THERAPEUTIC METHODS, which was filed on date even with the present application.

The following examples are for illustrative purposes only and are not intended to limit the scope of the invention or its various embodiments in any way.

### Example 1. Highly effective induction of immune responses by intra-lymphatic

### immunization.

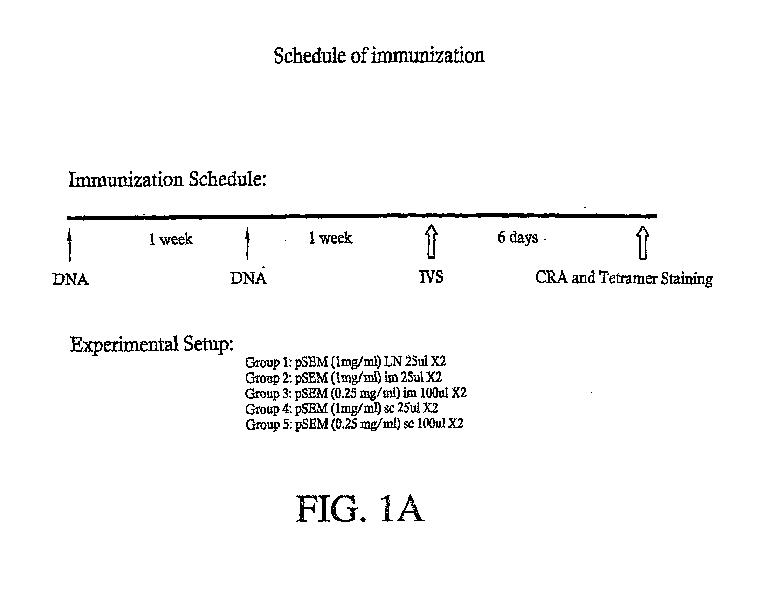

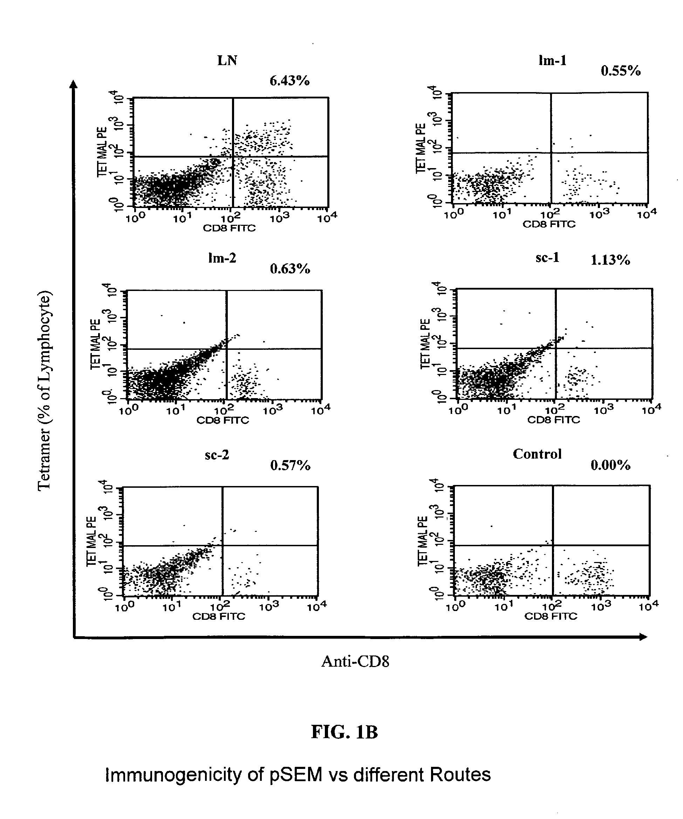

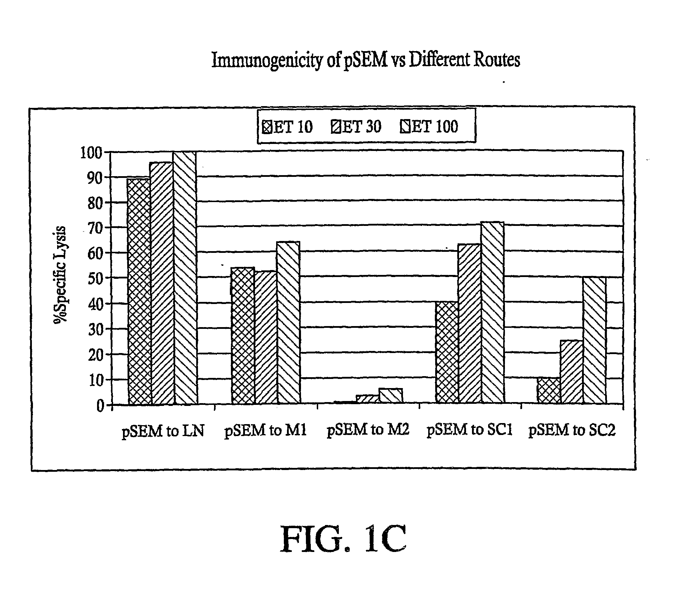

Mice carrying a transgene expressing a chimeric single-chain version of a human MHC class I (A*0201, designated "HHD"; see Pascolo et al. J. Exp. Med. 185(12):2043-51, 1997) were immunized by intranodal administration as follows. Five groups of mice (n=3) were immunized with plasmid expressing melan-A 26-35 A27L analogue (pSEM) for induction and amplified one week later, by employing different injection routes: subcutaneous (sc), intramuscular (im) and intralymphatic (in, using direct inoculation into the inguinal lymph nodes). The schedule of immunization and dosage is shown in Fig. 1A. One week after the amplification, the mice were sacrificed; the splenocytes were prepared and stained using tagged anti-CD8 mAbs and tetramers recognizing melan-A 26-35 -specific T cell receptors. Representative data are shown in Fig. 1B: while subcutaneous and intramuscular administration achieved frequencies of tetramer+CD8+ T cells around or less than 1%, intralymphatic administration of plasmid achieved a frequency of more than 6%. In addition, splenocytes were stimulated *ex vivo* with melan-A peptide and tested against ⁵¹Cr-labeled target cells (T2 cells) at various E:T ratios (Fig. 1C). The splenocytes from animals immunized by intralymph node injection showed the highest level of *in vitro* lysis at various E:T ratios, using this standard cytotoxicity assay.

### Example 2. Effects of the order in which different forms of immunogen are administered.

HHD mice were immunized by intranodal administration of plasmid (pSEM) or peptide (Mel A; ELAGIGILTV; SEQ ID NO:1) in various sequences. The immunogenic polypeptide encoded by pSEM is disclosed in U.S. Patent application 10/292,413 (Pub. No. 20030228634 A1) entitled EXPRESSION VECTORS ENCODING EPITOPES OF TARGET-ASSOCIATED ANTIGENS AND METHODS FOR THEIR DESIGN.

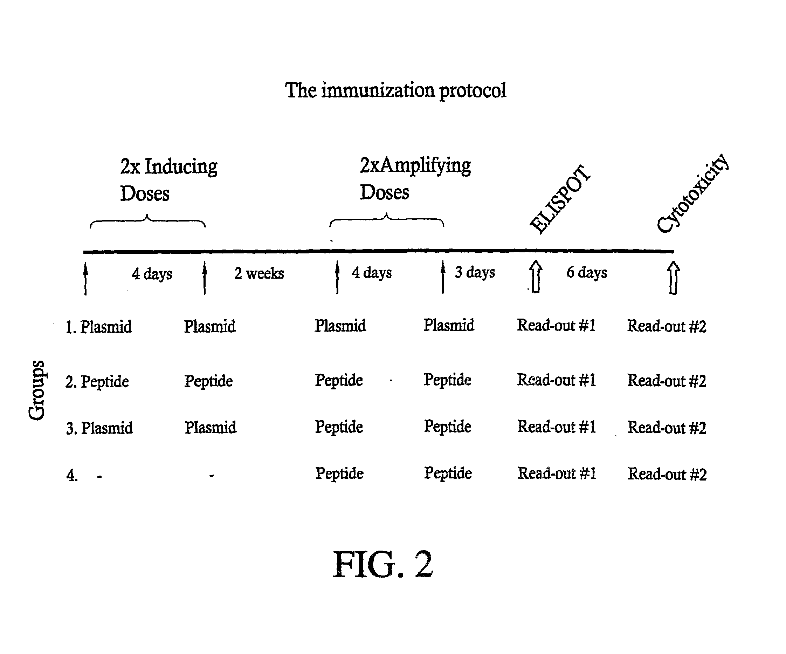

The protocol of immunization (Figure 2) comprised:
i) Induction Phase/Inducing doses: bilateral injection into the inguinal lymph nodes of 25 µl (microliters) of sterile saline containing either 25 µg (micrograms) of plasmid or 50 µg (micrograms) of peptide, at day 0 and day 4.
ii) Amplifying doses: as described above in Example 1 and initiated at 2 weeks after the completion of the induction phase.

The immune response was measured by standard techniques, after the isolation of splenocytes and *in vitro* stimulation with cognate peptide in the presence of pAPC. It is preferable that the profile of immune response be delineated by taking into account results stemming from multiple assays, facilitating assessment of various effector and regulatory functions and providing a more global view of the response. Consideration can be given to the type of assay used and not merely their number; for example, two assays for different proinflammatory cytokines is not as informative as one plus an assay for a chemokine or an immunosuppresive cytokine.

### Example 3. ELISPOT analysis of mice immunized as described in Example 2.

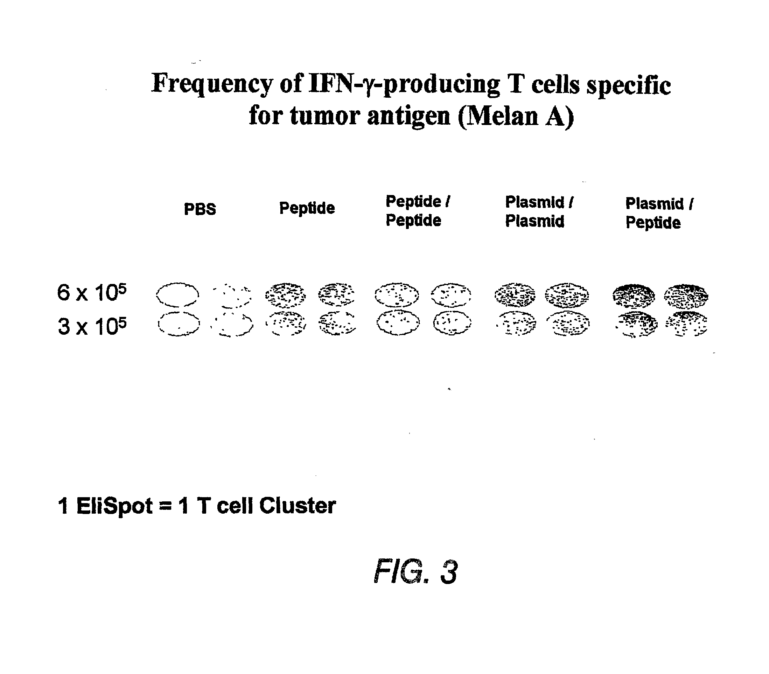

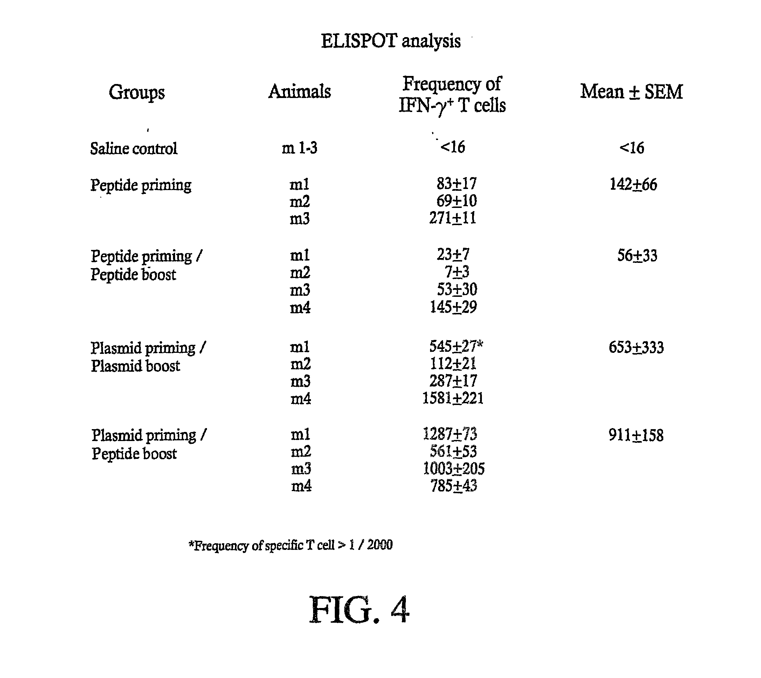

ELISPOT analysis measures the frequency of cytokine-producing, peptide-specific, T cells. Figure 3 presents representative examples in duplicates; and Figure 4 presents a summary of data expressed individually as number of cytokine producing cells /10⁶ responder cells. The results show that, in contrast to mice immunized with peptide, plasmid-immunized or plasmid-entrained / peptide-amplified mice developed elevated frequencies of IFN-γ (gamma)-producing T cells recognizing the melan-A peptide. Four out of four mice, entrained with plasmid and amplified with peptide, displayed frequencies in excess of 1/2000. In contrast, two out of four mice immunized throughout the protocol with plasmid, displayed frequencies in excess of 1/2000. None of the mice using only peptide as an immunogen mounted elevated response consisting in IFN-γ producing T cells. Indeed, repeated administration of peptide diminished the frequency of such cells, in sharp contrast to peptide administered after entrainment with plasmid.

### Example 4. Analysis of cytolytic activity of mice immunized as described in Example 2.

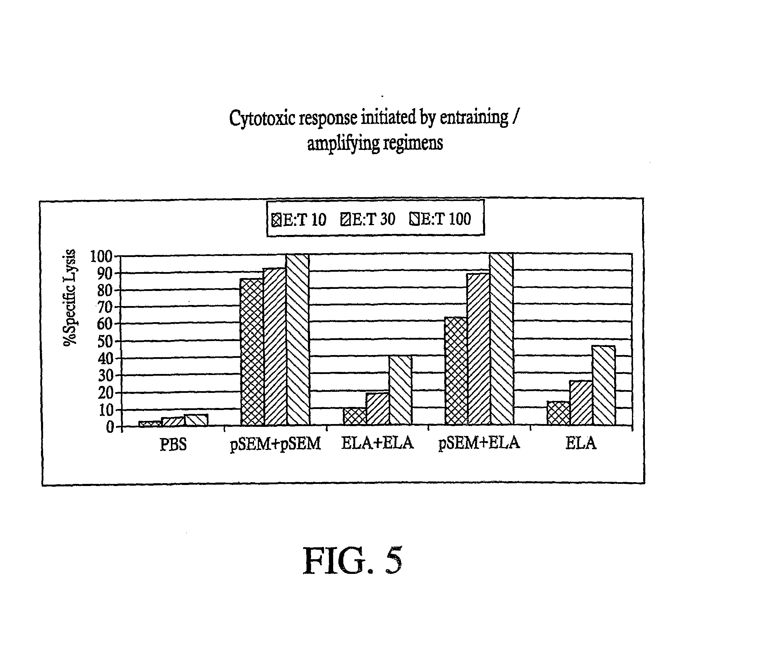

Pooled splenocytes were prepared (spleens harvested, minced, red blood cells lysed) from each group and incubated with LPS-stimulated, melan-A peptide-coated syngeneic pAPC for 7 days, in the presence of rIL-2. The cells were washed and incubated at different ratios with ⁵¹Cr-tagged T2 target cells pulsed with melan-A peptide (ELA), for 4 hours. The radioactivity released in the supernatant was measured using a γ (gamma)-counter. The response was quantified as % lysis = (sample signal - background) / (maximal signal - background) x 100, where background represents radioactivity released by target cells alone when incubated in assay medium, and the maximal signal is the radioactivity released by target cells lysed with detergent. Figure 5 illustrates the results of the above-described cytotoxicity assay. The levels of cytolytic activity achieved, after *in vitro* stimulation with peptide, was much greater for those groups that had received DNA as the inducing dose *in vivo* than those that had received peptide as the inducing dose. Consistent with the ELISPOT data above, induction of an immune response with a DNA composition led to stable, amplifiable effector function, whereas immunization using only peptide resulted in a lesser response, the magnitude of which further diminished upon repeated administration.

### Example 5. Cross-reactzvity

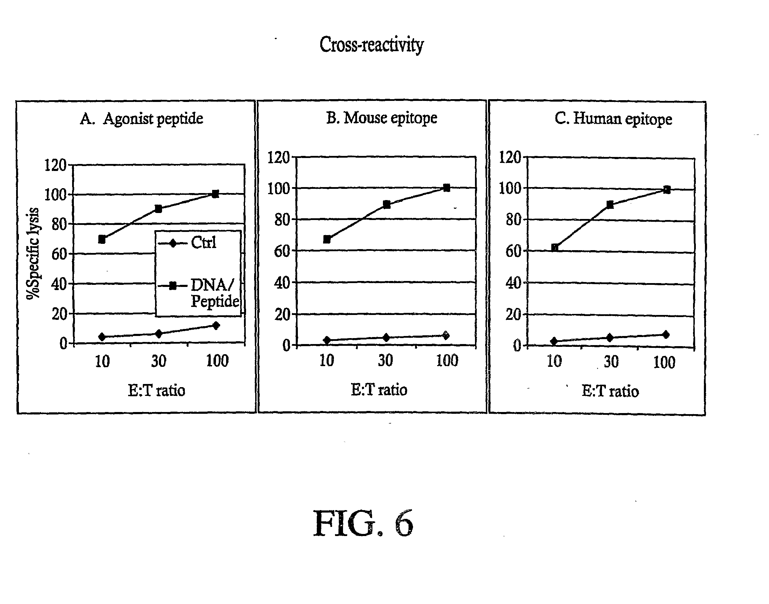

Splenocytes were prepared and used as above in Example 4 against target cells coated with three different peptides: the melan-A analogue immunogen and those representing the human and murine epitopes corresponding to it. As shown in Figure 6, similar cytolytic activity was observed on all three targets, demonstrating cross-reactivity of the response to the natural sequences.

### Example 6. Repeated administration of peptide to the lymph nodes induces immune

### deviation and regulatory T cells.

The cytokine profile of specific T cells generated by the immunization procedures described above (and in figure 2), was assessed by ELISA or Luminex®. (Luminex® analysis is a method to measure cytokine produced by T cells in culture in a multiplex fashion.) Seven-day supernatants of mixed lymphocyte cultures generated as described above were used for measuring the following biological response modifiers: MIP-1α RANTES and TGF-β (capture ELISA, using plates coated with anti-cytokine antibody and specific reagents such as biotin-tagged antibody, streptavidin-horse radish peroxidase and colorimetric substrate; R&D Systems). The other cytokines were measured by Luminex®, using the T1/T2 and the T inflammatory kits provided by specialized manufacturer (BD Pharmingen).

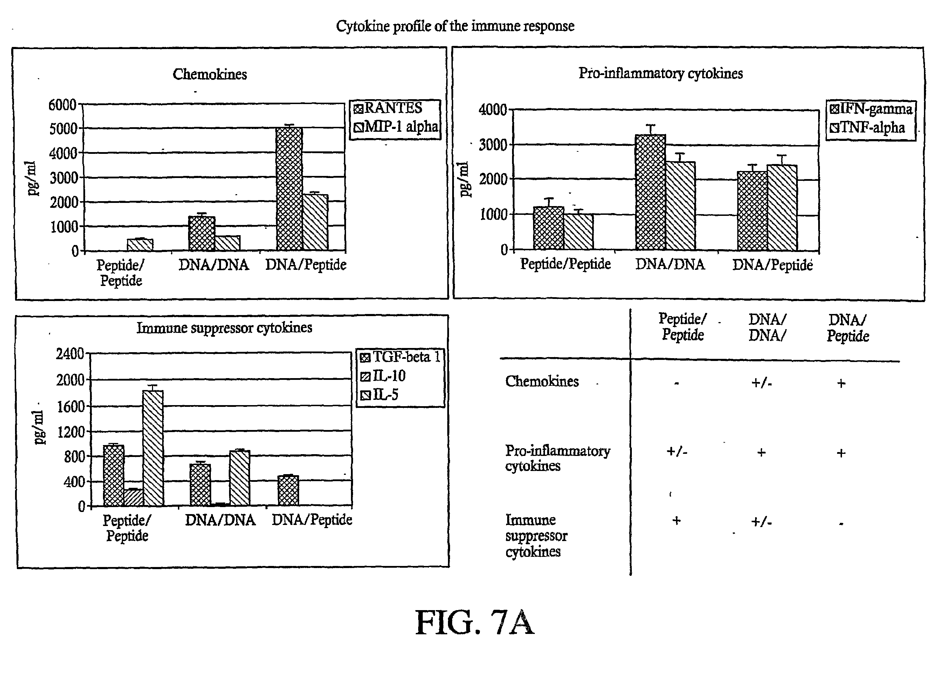

The data in Figure 7A compare the three different immunization protocols and show an unexpected effect of the protocol on the profile of immune response: whereas plasmid entrainment enabled the induction of T cells that secrete pro-inflammatory cytokines, repeated peptide administration resulted in generation of regulatory or immune suppressor cytokines such as IL-10, TGF-beta and IL-5. It should be appreciated that the immunization schedule used for the peptide-only protocol provided periodic rather than continuous presence of the epitope within the lymphatic system that instead prolongs the effector phase of the response. Finally, plasmid entrainment followed by peptide amplification resulted in production of elevated amounts of the T cell chemokines MIP-1α and RANTES. T cell chemokines such as MIP-1α and RANTES can play an important role in regulating the trafficking to tumors or sites of infection. During immune surveillance, T cells specific for target-associated antigens may encounter cognate ligand, proliferate and produce mediators including chemokines. These can amplify the recruitment of T cells at the site where the antigen is being recognized, permitting a more potent response. The data were generated from supernatants obtained from bulk cultures (means + SE of duplicates, two independent measurements).

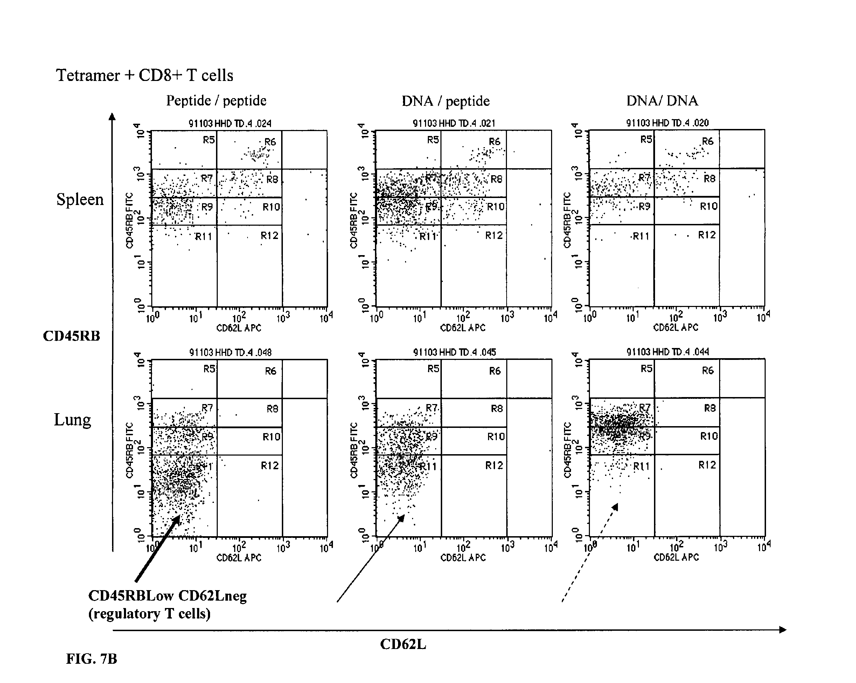

Cells were retrieved from the lung interstitial tissue and spleen by standard methods and stained with antibodies against CD8, CD62L and CD45RB, along with tetramer agent identifying Melan-A-specific T cells. The data in Fig. 7B represent gated populations of CD8+ Tetramer + T cells (y axis CD45RB and x axis CD62L).

Together, the results demonstrate immune deviation in animals injected with peptide only (reduced IFN-gamma, TNF-alpha production, increased IL-10, TGF-beta and IL-5, robust induction of CD62L- CD45Rblow CD8+ tetramer+ regulatory cells).

### Example 7. Highly effective induction of immune responses by alternating non-replicating plasmid (entrainment) with peptide (amplification) administered to the lymph node.

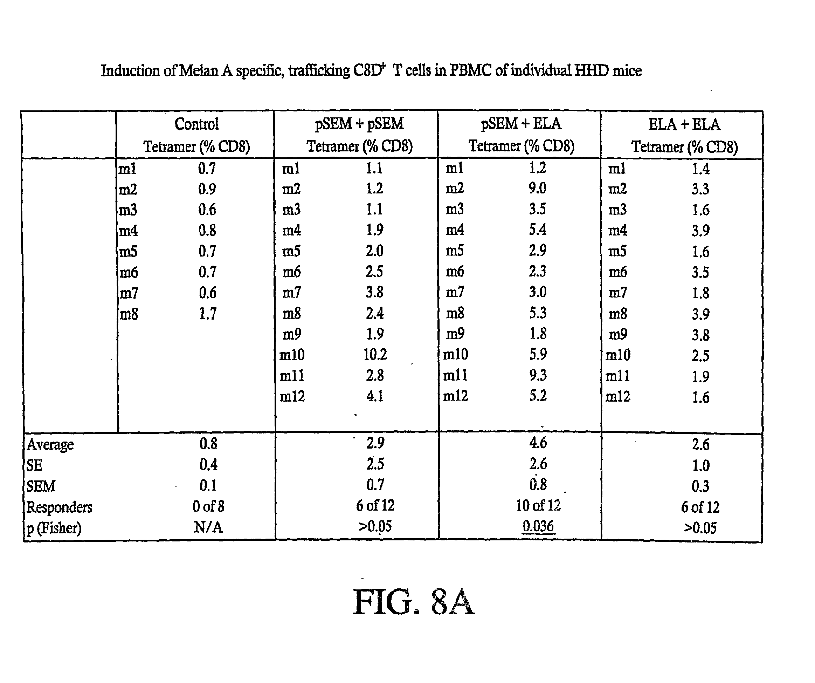

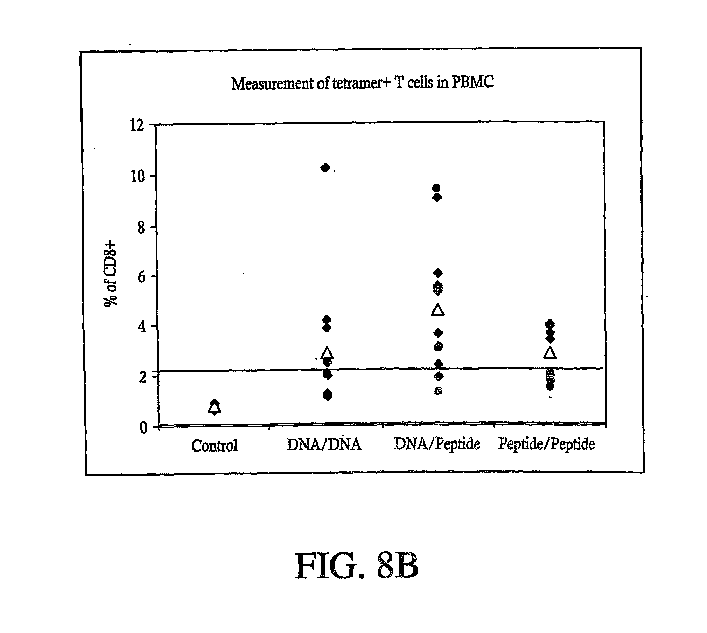

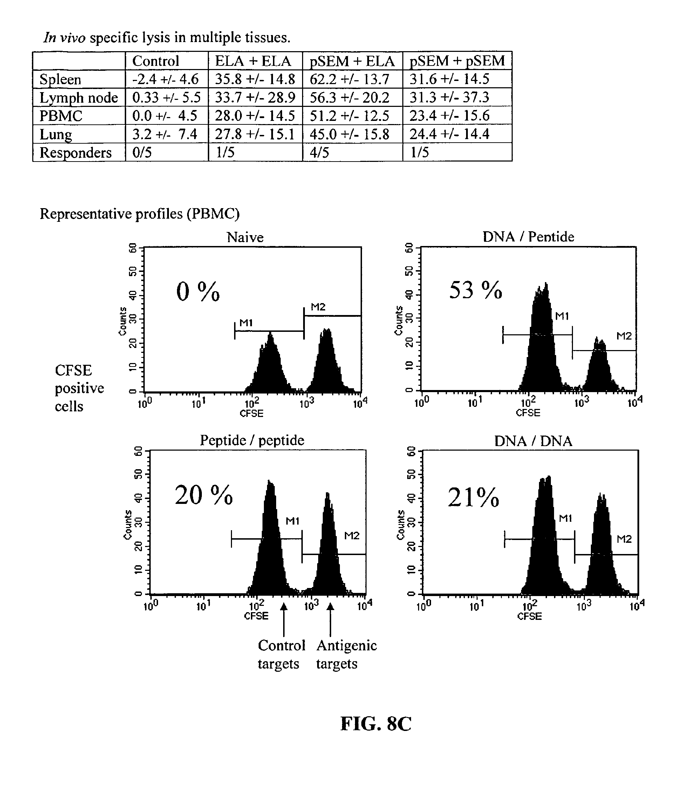

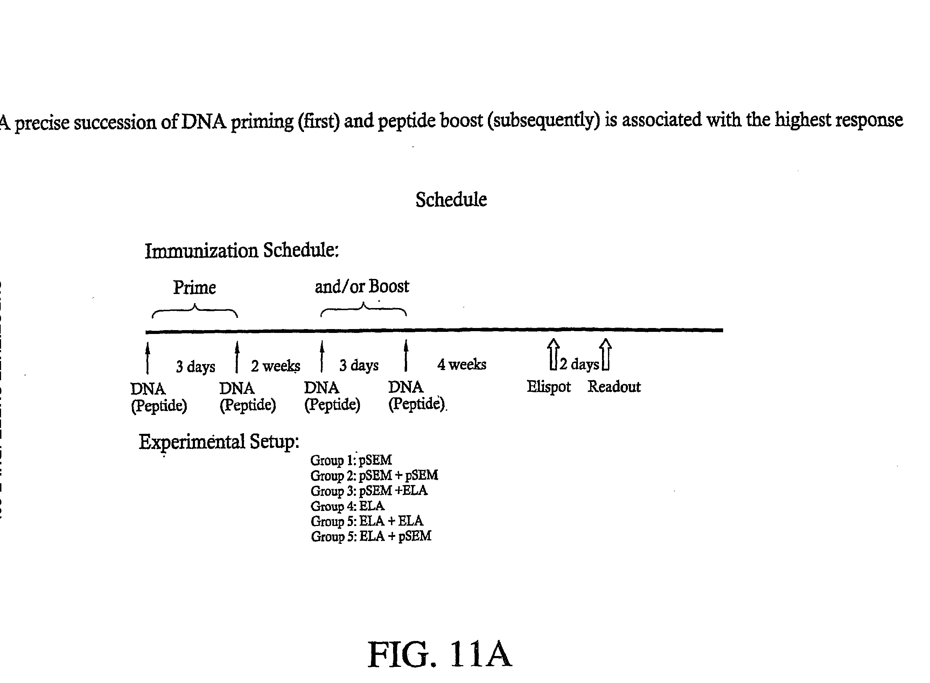

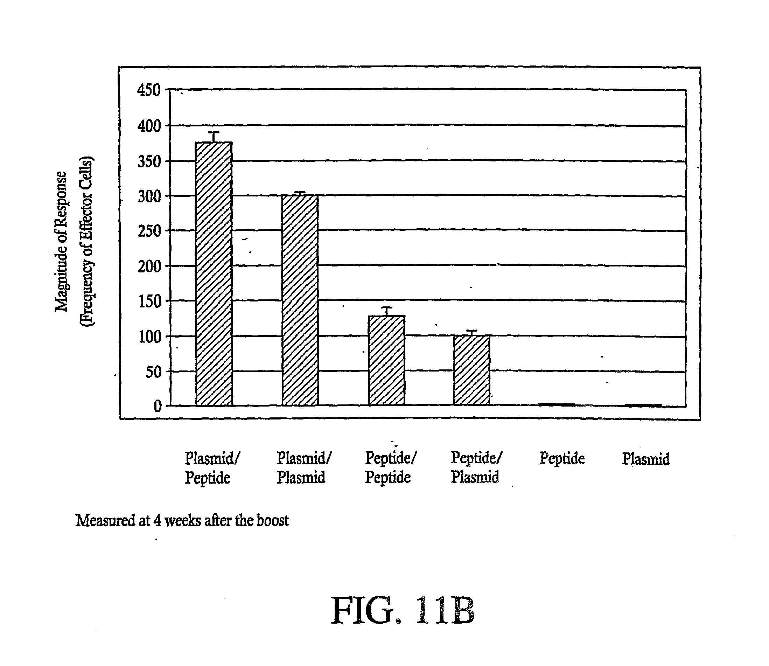

Three groups of HHD mice, transgenic for the human MHC class I HLA.A2 gene, were immunized by intralymphatic administration against the melan-A tumor associated antigen. Animals were primed (induced) by direct inoculation into the inguinal lymph nodes with either pSEM plasmid (25µg/lymph node) or ELA peptide (ELAGIGILTV, melan A 26-35 A27L analogue) (25µg/lymph node) followed by a second injection three days later. After ten days, the mice were boosted with pSEM or ELA in the same fashion followed by a final boost three days later to amplify the response (see Figure 11A for a similar immunization schedule), resulting in the following induce & amplify combinations: pSEM + pSEM, pSEM + ELA, and ELA + ELA (12 mice per group). Ten days later, the immune response was monitored using a melan-A specific tetramer reagent (HLA-A*0201 MART1 (ELAGIGILTV)-PE, Beckman Coulter). Individual mice were bled via the retro-orbital sinus vein and PBMC were isolated using density centrifugation (Lympholyte Mammal, Cedarlane Labs) at 2000rpm for 25 minutes. PBMC were co-stained with a mouse specific antibody to CD8 (BD Biosciences) and the melan-A tetramer reagent and specific percentages were determined by flow cytometery using a FACS caliber flow cytometer (BD). The percentages of melan-A specific CD8⁺ cells, generated by the different prime/boost combinations, are shown in Figures 8A and 8B. The plasmid-prime / peptide-boost group (pSEM + ELA) elicited a robust immune response with an average tetramer percentage of 4.6 between all the animals. Responder mice were defined to have tetramer percentages of 2 or greater which represented a value equivalent to the average of the unimmunized control group plus 3 times the standard deviation (SE). Such values are considered very robust responses in the art and can usually be achieved only by using replicating vectors. The pSEM + ELA immunization group contained 10 out of 12 mice that were found to be responders and this represented a statistically significant difference as compared to the control group (p (Fisher) = 0.036). The other two immunization series, pSEM + pSEM and ELA + ELA, yielded 6 out of 12 responders but had p values greater than 0.05 rendering them less statistically significant. To measure the immunity of these mice, animals were challenged with peptide coated target cells *in vivo.* Splenocytes were isolated from littermate control HHD mice and incubated with 20µg/mL ELA peptide for 2 hours. These cells were then stained with CFSE^{hi} fluorescence (4.0µM for 15 minutes) and intravenously co-injected into immunized mice with an equal ratio of control splenocytes that had not been incubated with peptide, stained with CFSE^{lo} fluorescence (0.4µM). Eighteen hours later the specific elimination of target cells was measured by removing spleen, lymph node, PBMC, and lung from challenged animals (5 mice per group) and measuring CFSE fluorescence by flow cytometry. The results are shown in Figure 8C. In the pSEM + ELA prime/boost group, 4 out of 5 mice demonstrated a robust immune response and successfully cleared roughly 50% of the targets in each of the tissues tested. Representative histograms for each experimental groups are showed as well (PBMC).

### Example 8. Peptide boost effectively reactivates the immune memory cells in animals induced with DNA and rested until tetramer levels were close to baseline.

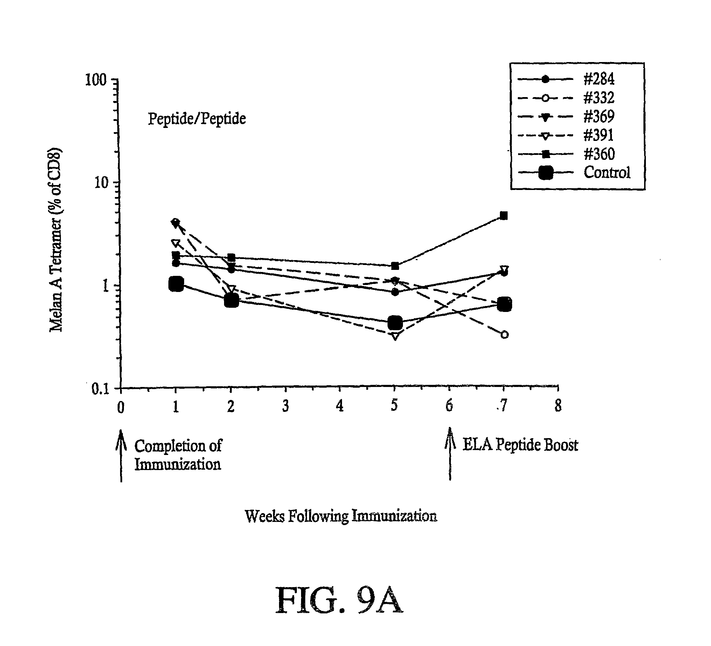

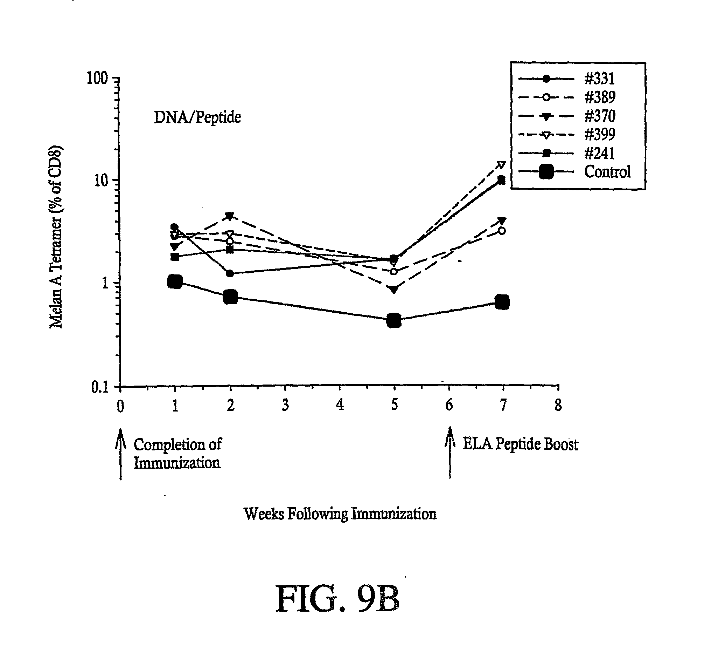

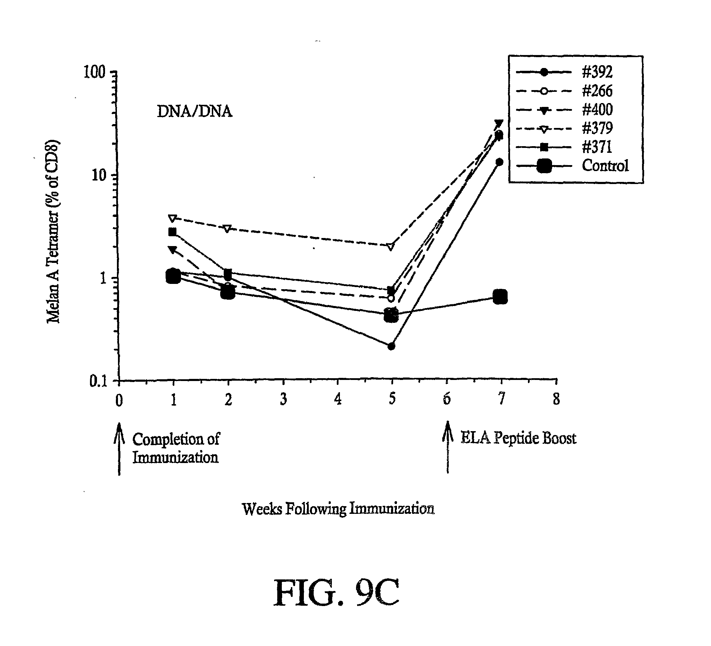

Melan-A tetramer levels were measured in mice (5 mice per group) following immunization, as described in Figure 9A. By 5 weeks after completion of the immunization schedule, the tetramer levels had returned close to baseline. The animals were boosted at 6 weeks with ELA peptide to determine if immune responses could be restored. Animals receiving prior immunizations of pSEM plasmid (DNA/DNA, Figure 9C) demonstrated an unprecedented expansion of melan-A specific CD8⁺ T cells following the ELA amplification, with levels in the range of greater than 10%. On the other hand, animals receiving prior injections of ELA peptide (Figure 9A) derived little benefit from the ELA boost as indicated by the lower frequency of tetramer staining cells. Mice that received DNA followed by peptide as the initial immunization exhibited a significant, but intermediate, expansion upon receiving the peptide amplication, as compared to the other groups. (Figure 9B). These results clearly demonstrate a strong rationale for a DNA/DNA-entrainment and peptide-amplification immunization strategy.

### Example 9. Optimization of immunization to achieve high frequencies of specific T cells in lymphoid and non-lymphoid organs.

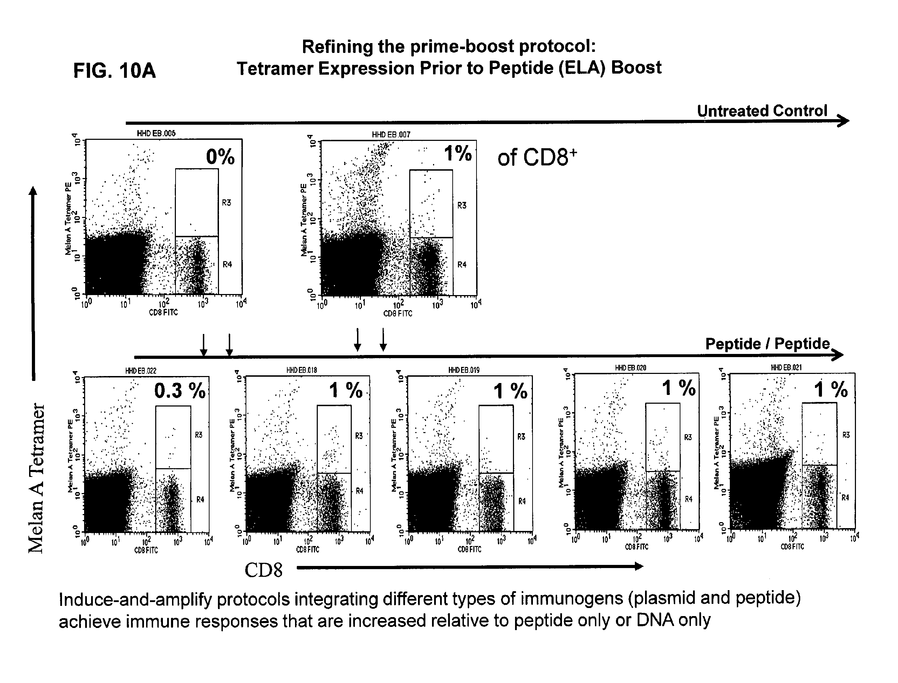

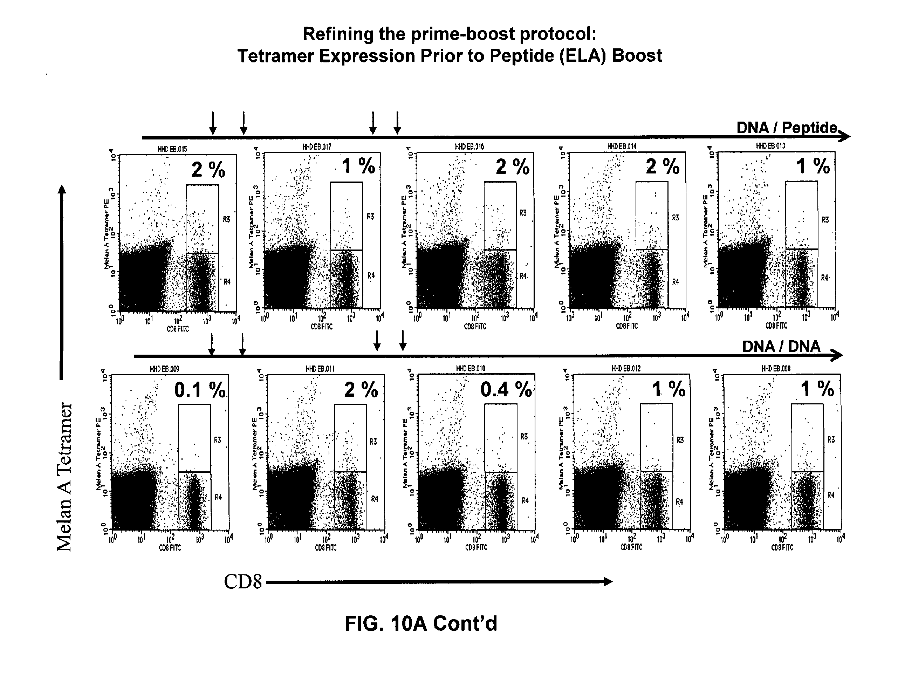

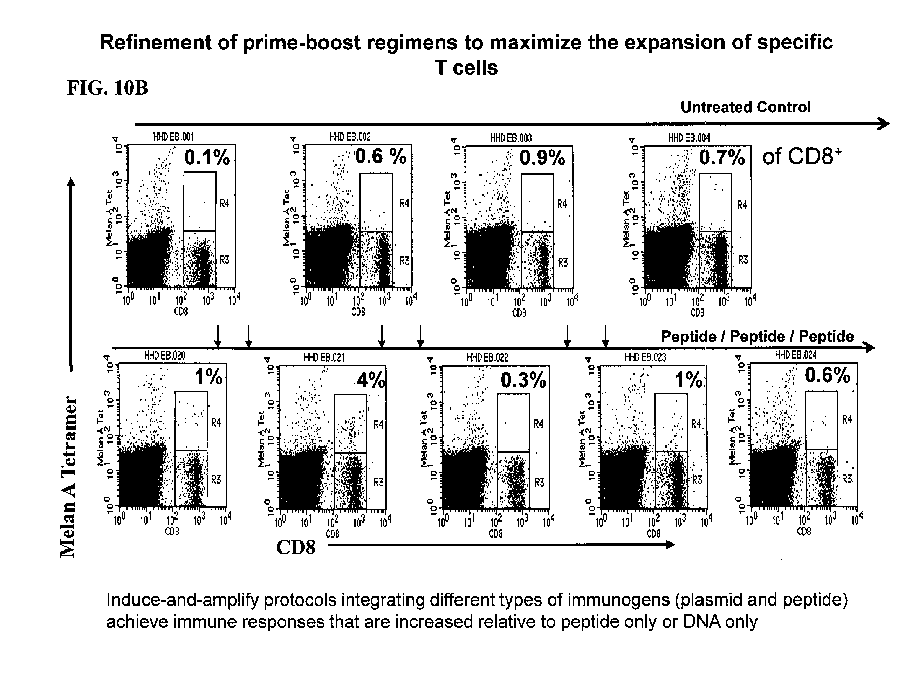

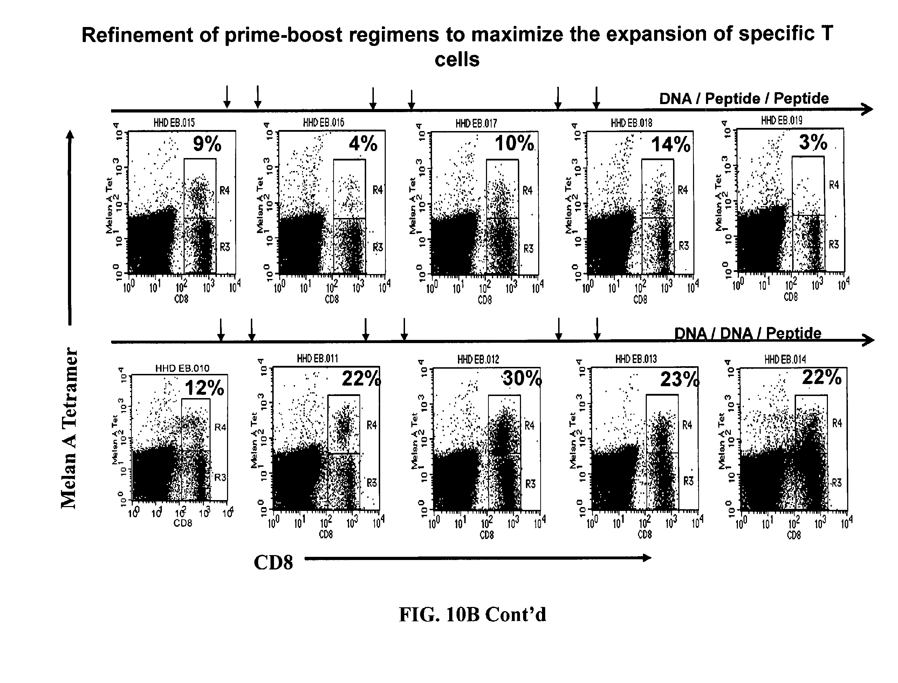

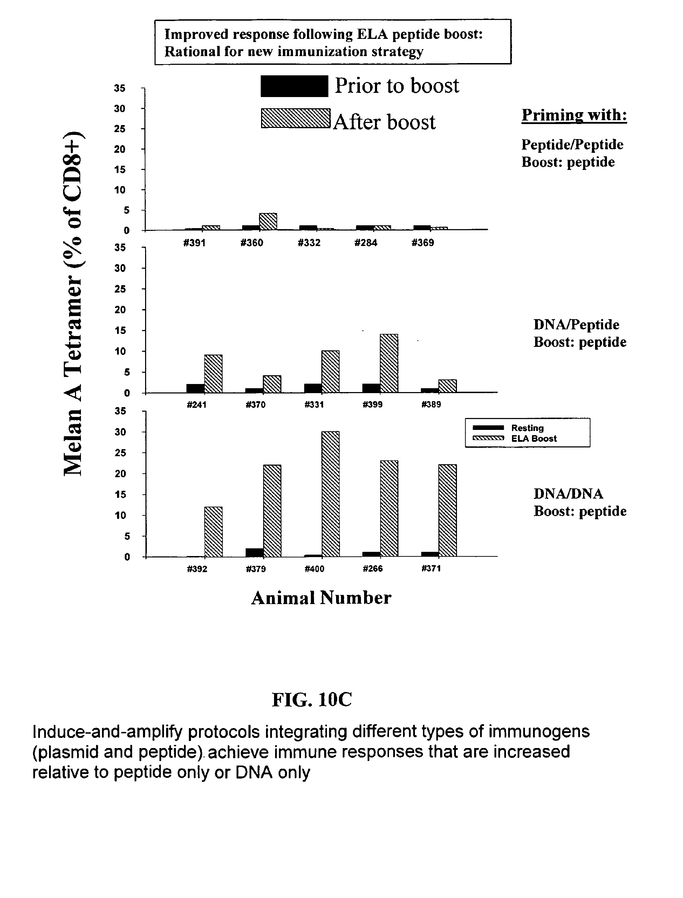

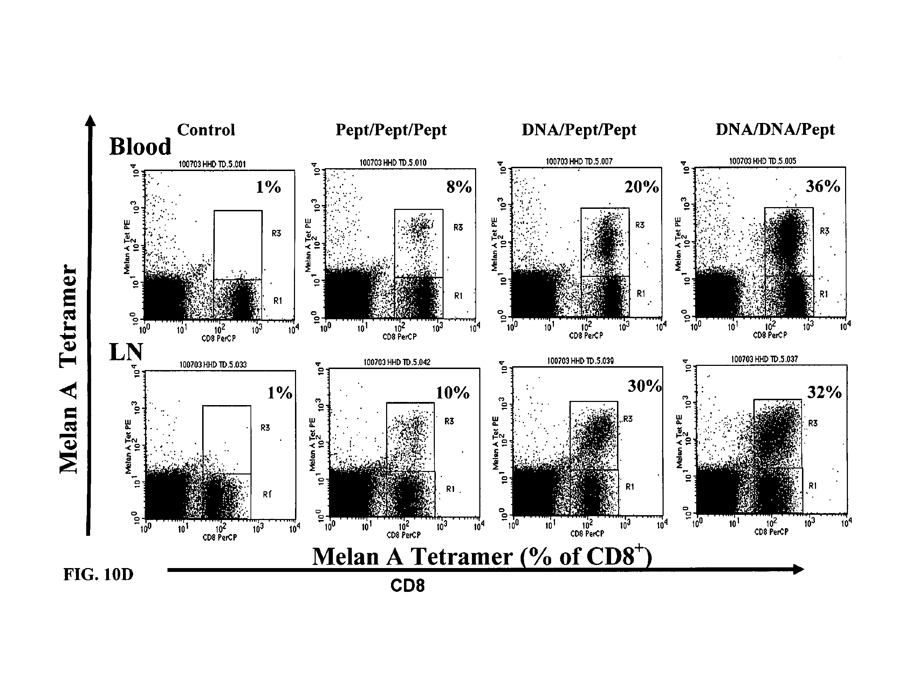

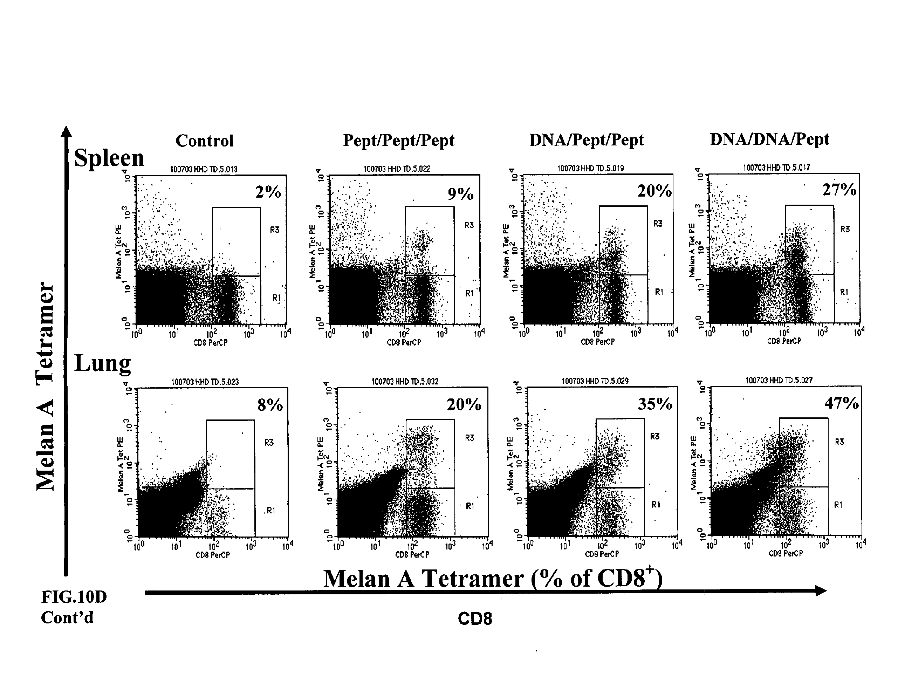

As described in Figure 9A-C, mice that were subjected to an entraining immunization with a series of two clusters of plasmid injections followed by amplification with peptide yielded a potent immune response. Further evidence for this is shown in Figure 10A-C which illustrate the tetramer levels prior to (Figure 10A) and following peptide administration (Figure 10B). Tetramer levels in individual mice can be clearly seen and represent up to 30% of the total CD8⁺ population of T cells in mice receiving the DNA/DNA/Peptide immunization protocol. These results are summarized in the graph in Figure 10C. In addition, high tetramer levels are clearly evident in blood, lymph node, spleen, and lung of animals receiving this refined immunization protocol (Figure 10D).

### Example 10. A precise administration sequence of plasmid and peptide immunogen determines the magnitude of immune response.

Six groups of mice (n=4) were immunized with plasmid expressing melan-A 26-35 A27L analogue (pSEM) or melan-A peptide using priming and amplificationt by direct inoculation into the inguinal lymph nodes. The schedule of immunization is shown in Fig. 11A (doses of 50µg of plasmid or peptide / lymph node, bilaterally). Two groups of mice were initiated using plasmid and amplified with plasmid or peptide. Conversely, two groups of mice were initiated with peptide and amplified with peptide or plasmid. Finally, two groups of control mice were initiated with either peptide or plasmid but not amplified. At four weeks after the last inoculation, the spleens were harvested and splenocyte suspensions prepared, pooled and stimulated with melan-A peptide in ELISPOT plates coated with anti-IFN-γ antibody. At 48 hours after incubation, the assay was developed and the frequency of cytokine-producing T cells that recognized melan-A was automatically counted. The data were represented in Fig 5B as frequency of specific T cells / 1 million responder cells (mean of triplicates + SD). The data showed that reversing the order of initiating and amplifying doses of plasmid and peptide has a substantial effect on the overall magnitude of the response: while plasmid entrainment followed by peptide amplification resulted in the highest response, initiating doses of peptide followed by plasmid amplification generated a significantly weaker response, similar to repeated administration of peptide.

### Example 11. Correlation of immune responses with the protocol of immunization and in vivo efficacy--manifested by clearing of target cells within lymphoid and non-lymphoid organs.

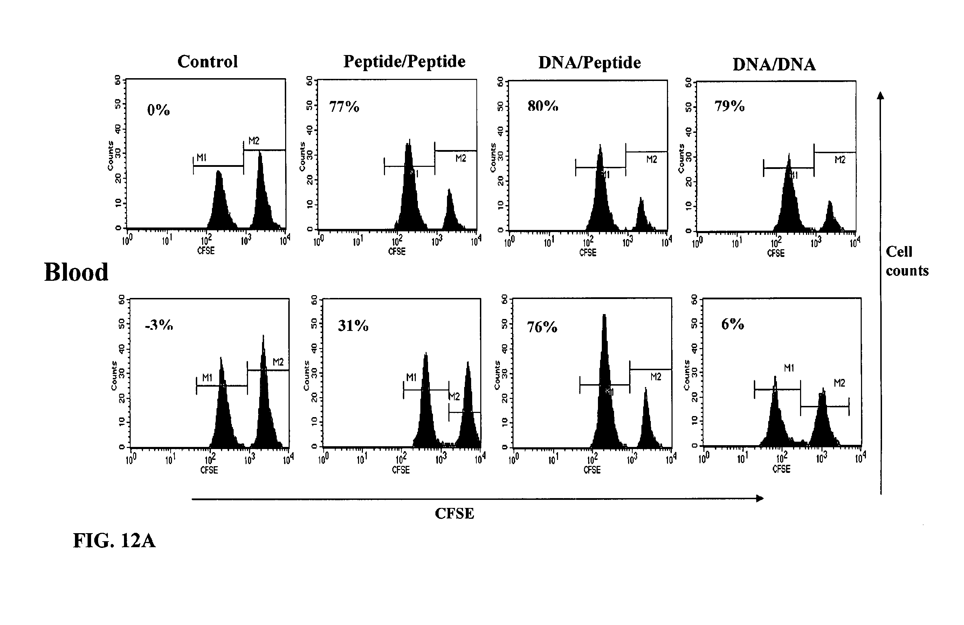

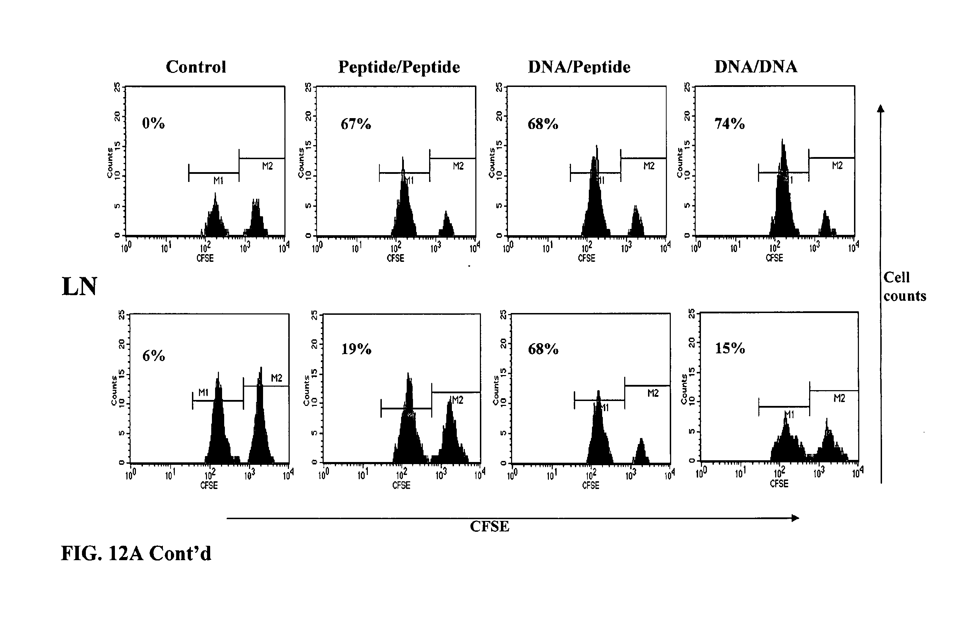

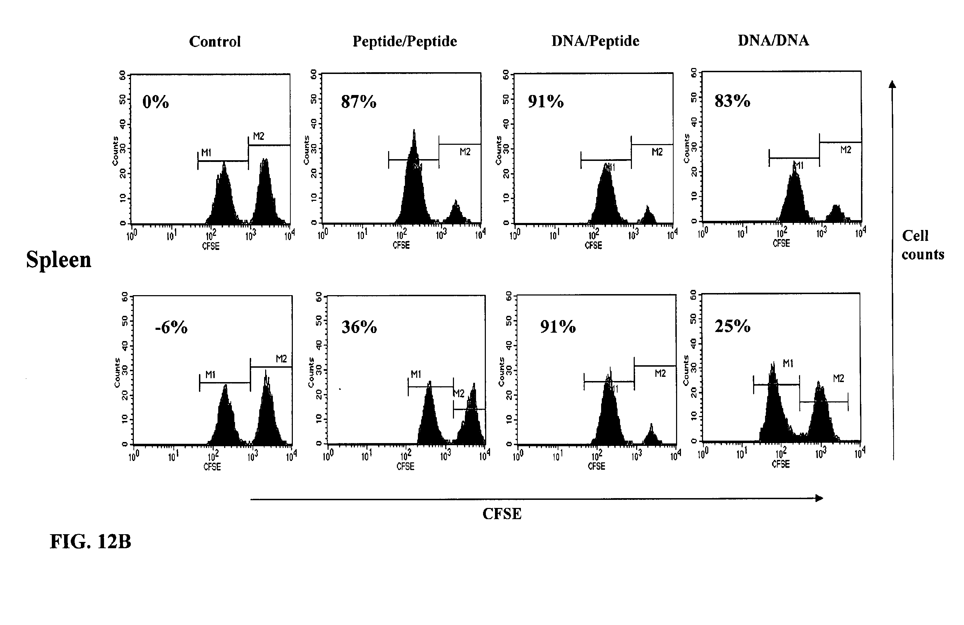

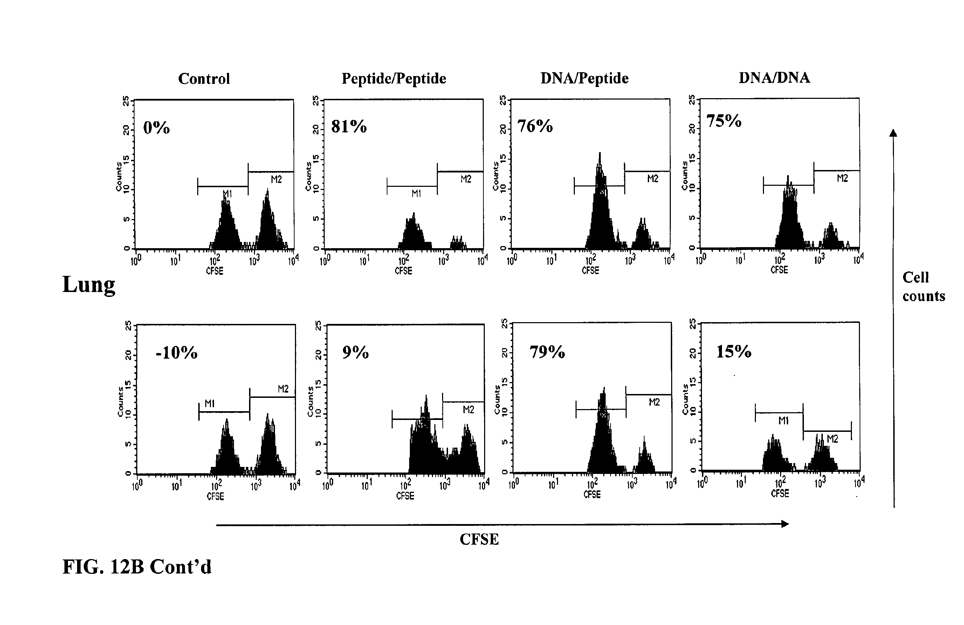

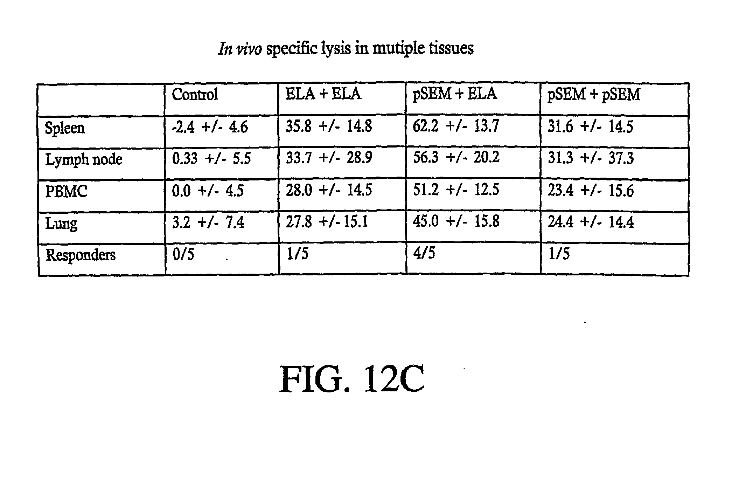

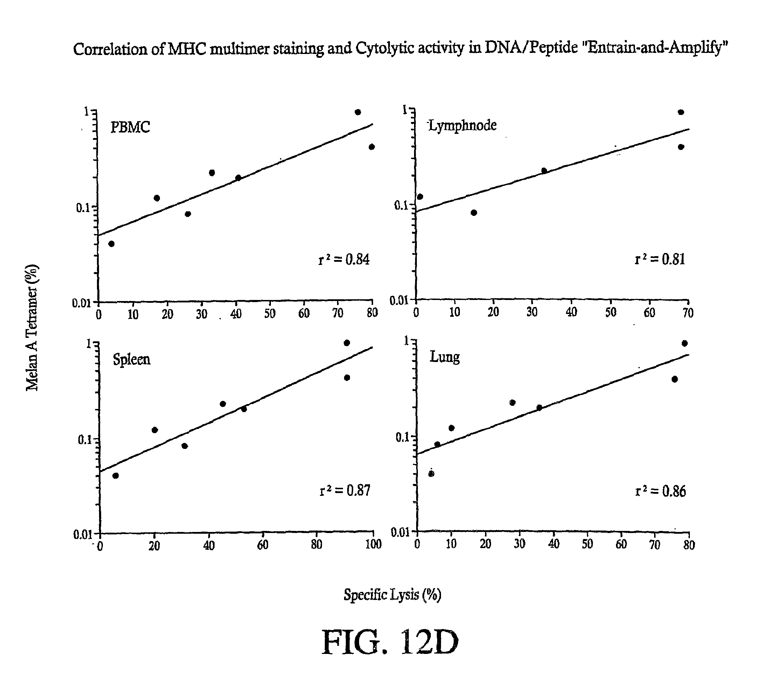

To evaluate the immune response obtained by the entrain-and-amplify protocol, 4 groups of animals (n=7) were challenged with melan-A coated target cells *in vivo.* Splenocytes were isolated from littermate control HHD mice and incubated with 20µg/mL ELA peptide for 2 hours. These cells were then stained with CFSE^{hi} fluorescence (4.0µM for 15 minutes) and intravenously co-injected into immunized mice with an equal ratio of control splenocytes stained with CFSE^{lo} fluorescence (0.4µM). Eighteen hours later the specific elimination of target cells was measured by removing spleen, lymph node, PBMC, and lung from challenged animals and measuring CFSE fluorescence by flow cytometry. Figures 12A and 12B show CFSE histogram plots from tissues of unimmunized control animals or animals receiving a immunization protocol of peptide/peptide, DNA/peptide, or DNA/DNA (two representative mice are shown from each group). The DNA-entrain/peptide-amplify group demonstrated high levels of specific killing of target cells in lymphoid as well as non-lymphoid organs (Figure 12C) and represented the only immunization protocol that demonstrated a specific correlation with tetramer levels (Figure 12D, r² = 0.81 or higher for all tissues tested).

### Example 12. Clearance of human tumor cells in animals immunized by the refined entrain-and -amplify protocol.

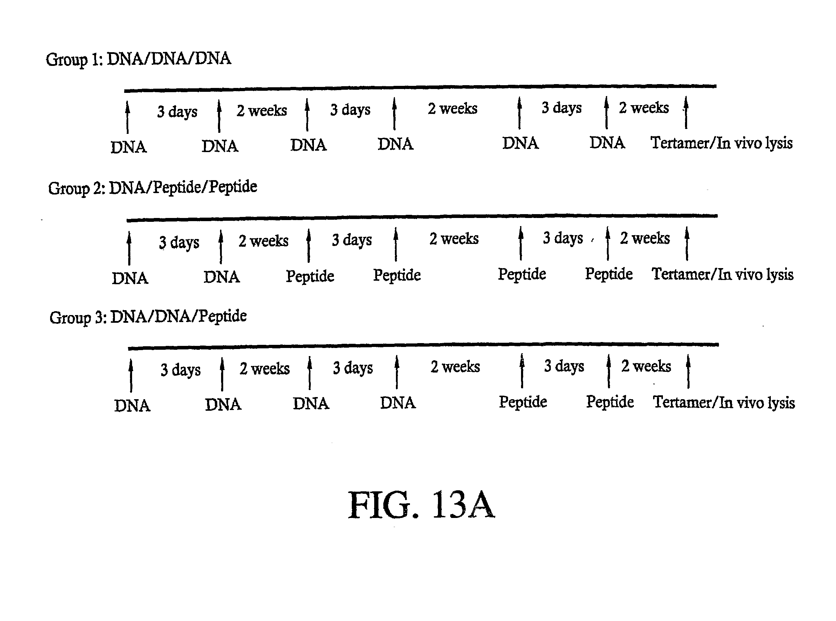

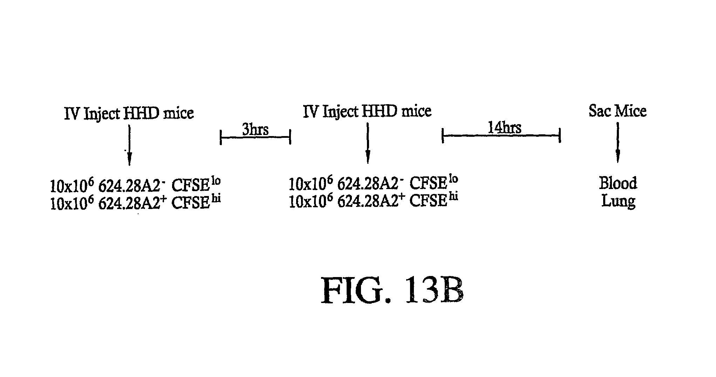

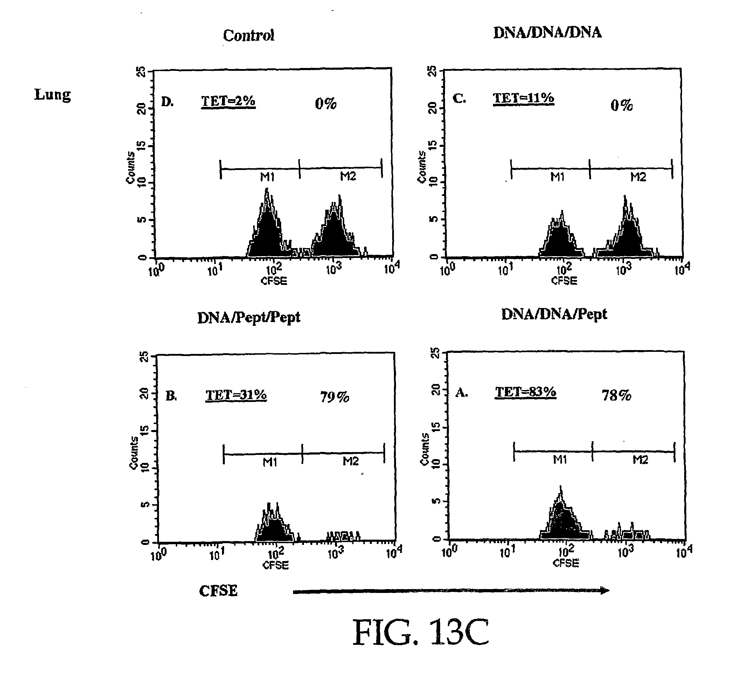

Immunity to the melan-A antigen was further tested by challenging mice with human melanoma tumor cells following immunization with the refined protocol. Figure 13A shows the refined immunization strategy employed for the 3 groups tested. Immunized mice received two intravenous injections of human target cells, 624.38 HLA.A2⁺, labeled with CFSE^{hi} fluorescence mixed with an equal ratio of 624.28 HLA.A2-control cells labeled with CFSE^{lo} as illustrated in Figure 13B. Fourteen hours later, the mice were sacrificed and the lungs (the organ in which the human targets accumulate) were analyzed for the specific lysis of target cells by flow cytometry. Figure 13C shows representative CFSE histogram plots derived from a mouse, from each group. DNA-entrainment followed by a peptide-amplification clearly immunized the mice against the human tumor cells as demonstrated by nearly 80% specific killing of the targets in the lung. The longer series of DNA-entrainment injections also led to a further increased frequency of CD8⁺ cells reactive with the melan-A tetramer.

### Example 13. DNA-entraining, peptide-amplification strategy results in robust immunity against an SSX2-derived epitope, KASEKIFYV (SSX2₄₁₋₄₉).

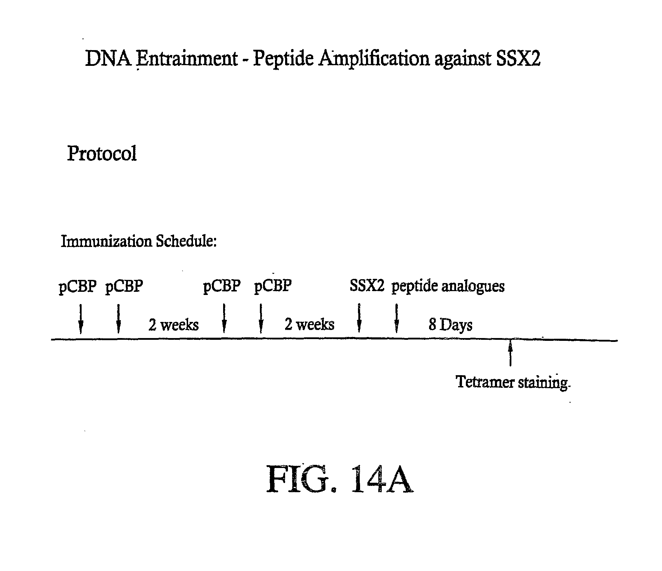

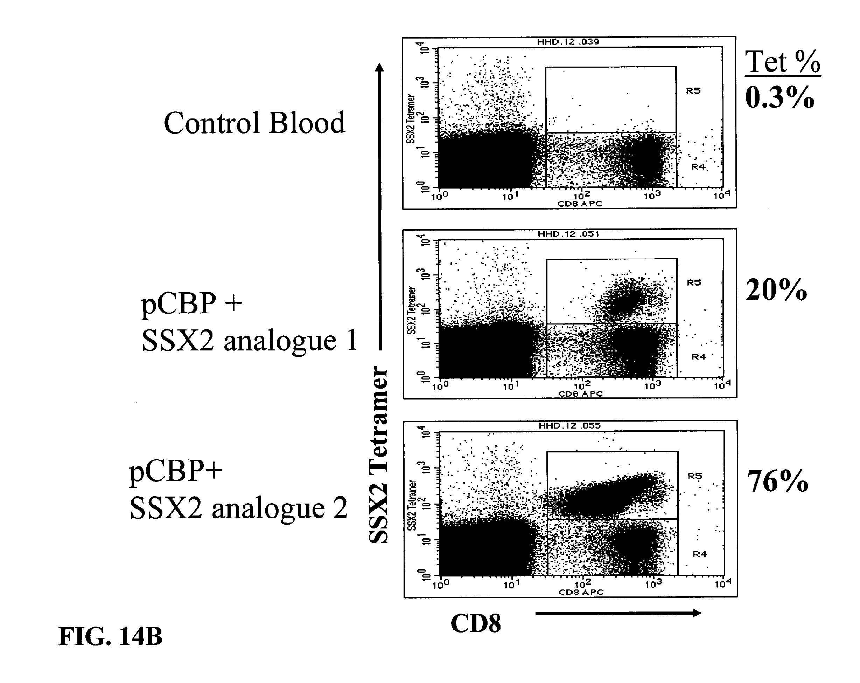

Animals immunized against the SSX2 tumor associated antigen using the immunization schedule defined in Figure 14A, demonstrated a robust immune response. Figure 14B shows representative tetramer staining of mice primed (entrained) with the pCBP plasmid and boosted (amplified) with either the SSX2₄₁₋₄₉ K41F or K41Y peptide analogue. These analogues are cross-reactive with T cells specific for the SSX2₄₁₋₄₉ epitope. These examples illustrate that the entrain-and-amplify protocol can elicit a SSX2 antigen specificity that approaches 80% of the available CD8 T cells. The pCBP plasmid and principles of its design are disclosed in US Patent Application No. 10/292,413 (Pub. No. 20030228634 A1) entitled EXPRESSION VECTORS ENCODING EPITOPES OF TARGET-ASSOCIATED ANTIGENS AND METHODS FOR THEIR DESIGN. Additional methodology, compositions, peptides, and peptide analogues are disclosed in U.S. Provisional Application No. / , (Attorney Docket MANNK.038PR), filed on the same date as the instant application, entitled "SSX-2 PEPTIDE ANALOGS;". Further methodology, compositions, peptides, and peptide analogues are disclosed in U.S. Provisional Application No. / , (Attorney Docket MANNK.039PR), filed on the same date as the instant application, entitled "NY-ESO PEPTIDE ANALOGS;".

### Example 14. The Entrain-and-Amplify strategy can be used to elicit immune responses against epitope located on different antigens simultaneously.

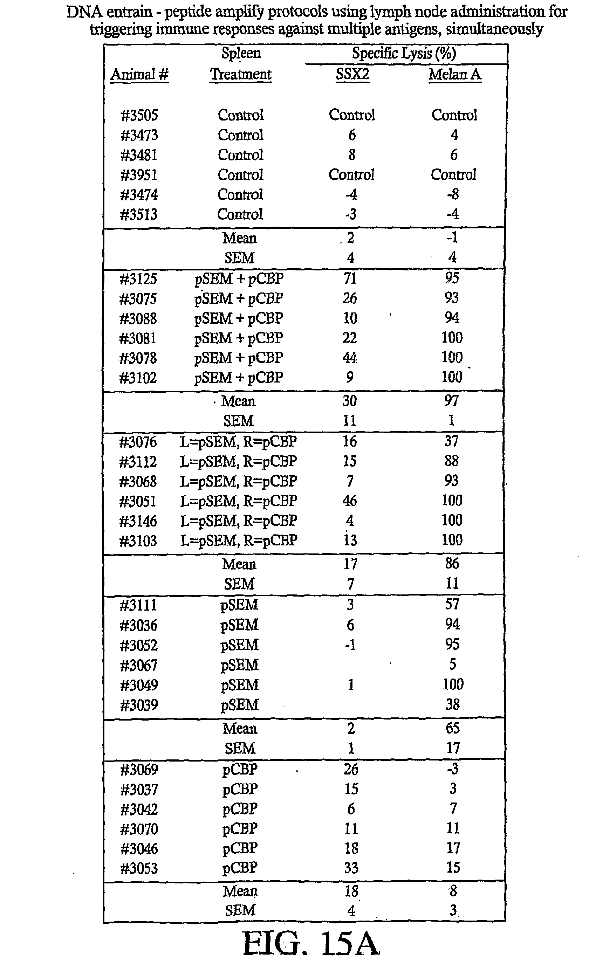

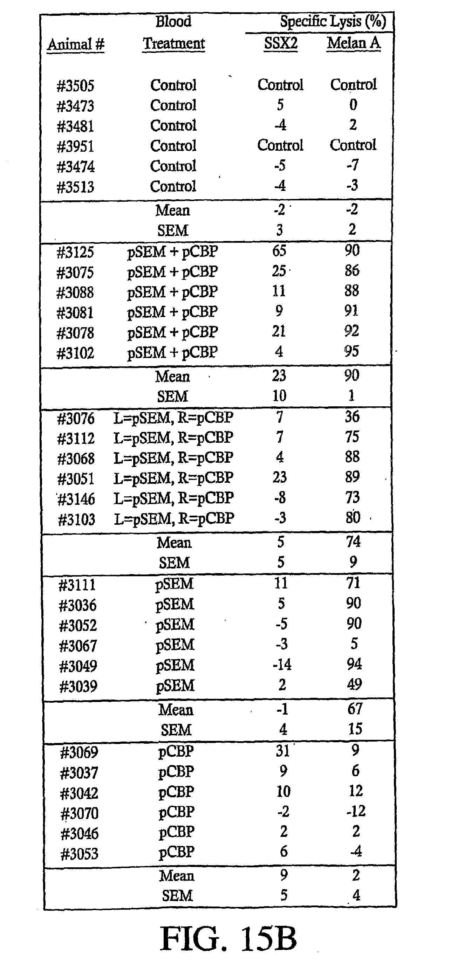

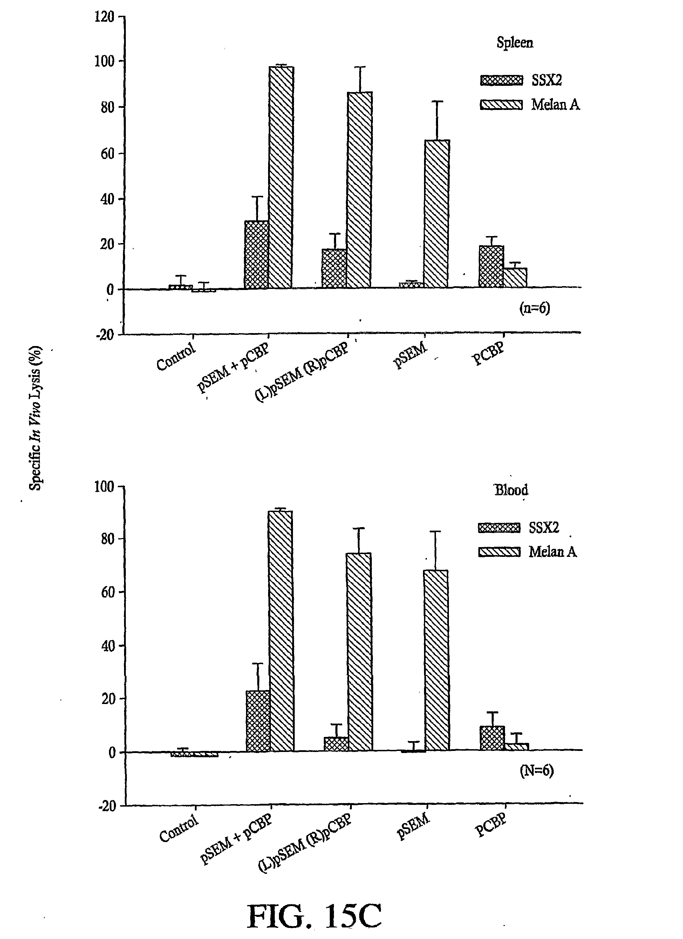

Four groups of HHD mice (n=6) were immunized via intra lymph node injection with either pSEM alone; pCBP alone; pSEM and pCBP as a mixture; or with pSEM in the left LN and pCBP in the right LN. These injections were followed 10 days later with either an ELA or SSX2 peptide boost in the same fashion. All immunized mice were compared to unimmunized controls. The mice were challenged with HHD littermate splenocytes coated with ELA or SSX2 peptide, employing a triple peak CFSE *in vivo* cytotoxicity assay that allows the assessment of the specific lysis of two antigen targets simultaneously. Equal numbers of control-CFSE^{lo}, SSX2-CFSE^{med}, and ELA-CFSE^{hi} cells were intravenously infused into immunized mice, and 18 hours later the mice were sacrificed and target cell elimination was measured in the spleen (Fig. 15A) and blood (Fig. 15B) by CFSE fluorescence using a flow cytometer. Figures 15A and 15B show the percent specific lysis of the SSX2 and Melan-A antigen targets from individual mice and Figure 15C summarizes the results in a bar graph format. Immunizing the animals with a mixture of two vaccines generated immunity to both antigens and resulted in the highest immune response, representing an average SSX2 percent specific lysis in spleen of 30+/-11 and 97+/-1 for Melan-A.

### Example 15. Repeated cycles of DNA entrainment and peptide amplification achieve and maintain strong immunity.

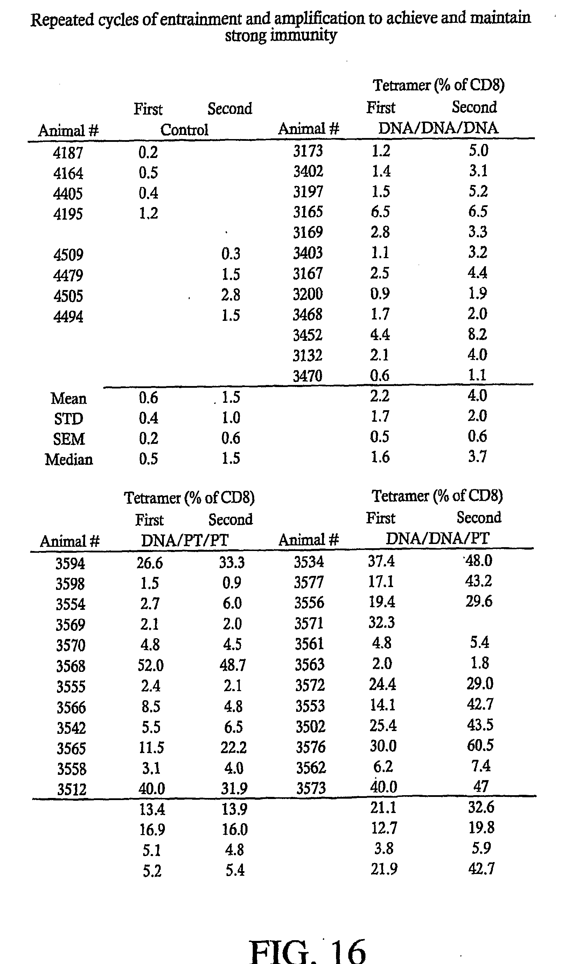

Three groups of animals (n=12) received two cycles of the following immunization protocols: DNA/DNA/DNA; DNA/peptide/peptide; or DNA/DNA/peptide. Melan-A tetramer levels were measured in the mice following each cycle of immunization and are presented in Figure 16. The initial DNA/DNA/peptide immunization cycle resulted in an average of 21.1+/-3.8 percent tetramer⁺ CD8⁺ T cells-nearly 2 fold higher than the other two groups. Following the second cycle of entrain-and-amplify immunization the average tetramer percentage for the DNA/DNA/peptide group increased by 54.5% to 32.6+/-5.9-2.5-fold higher than the DNA/peptide/peptide levels and 8.25-fold higher than the DNA/DNA/DNA group levels. In addition, under these conditions, the other immunization schedules achieved little increase in the frequency of tetramer positive T cells.

### Example 16. Long-lived memory T cells triggered by immune inducing and amplifying regimens, consisting in alternating plasmid and peptide vectors.

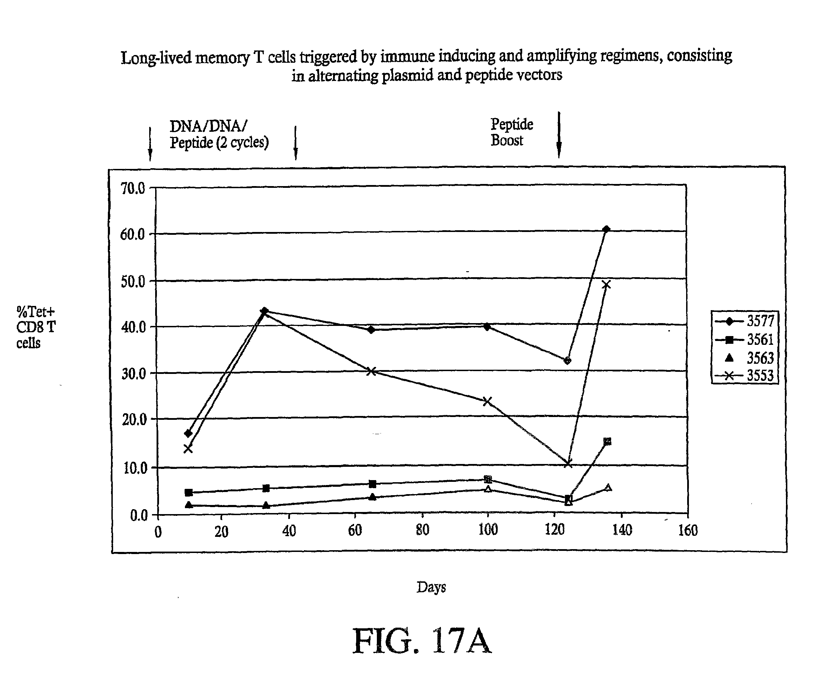

Four HHD transgenic animals (3563, 3553, 3561 and 3577) received two cycles of the following entrain-and-amplify protocol: DNA/DNA/peptide. The first cycle involved immunization on days -31, -28, -17, -14, -3, 0; the second cycle involved immunizations on day 14, 17, 28, 31, 42 and 45. Mice were boosted with peptide on day 120. Melan-A tetramer levels were measured in the mice at 7-10 days following each cycle of immunization and periodically until 90 days after the second immunization cycle. The arrows in the diagram correspond to the completion of the cycles. (Figure 17A). All four animals mounted a response after the last boost (amplification), demonstrating persistence of immune memory rather than induction of tolerance.

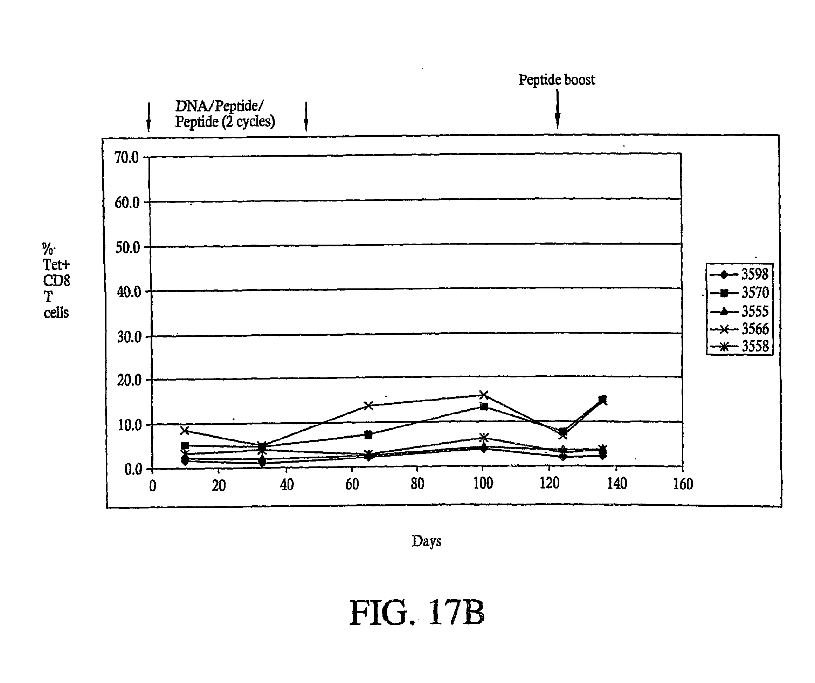

Five HHD transgenic animals (3555, 3558, 3566, 3598 and 3570) received two cycles of the following entrain-and-amplify protocol: DNA/peptide/peptide. As before, the first cycle consisted in immunization on days -31, -28, -17, -1,4, -3, 0; the second cycle consisted in immunizations on day 14, 17, 28, 31, 42 and 45.. Mice were boosted with peptide on day 120. Melan-A tetramer levels were measured in the mice at 7-10 days following each cycle of immunization and periodically until 90 days after the second immunization cycle (Figure 17B). By comparison this entrain-and-amplify protocol substituting peptide for the later DNA injections in each cycle resulted, in this experiment, in diminished immune memory or reduced responsiveness.

### Example 17. Long-lived memory T cells with substantial expansion capability are generated by intranodal DNA administration.

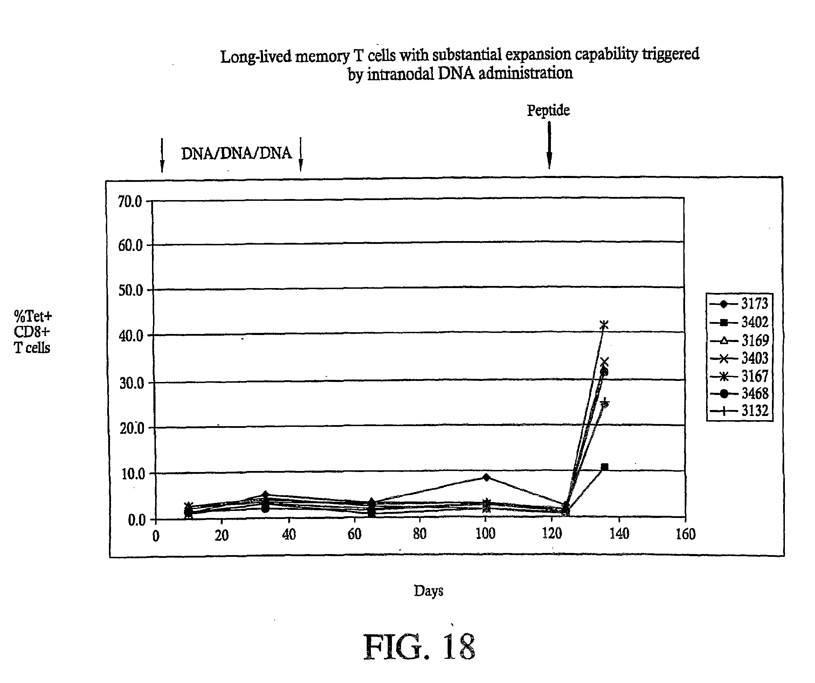

Seven HHD transgenic animals received two cycles of the following immunization protocol: DNA/DNA/DNA. The first cycle involved immunization on days - 31, -28, -17, -14, -3, 0; the second cycle involved immunizations on day 14, 17, 28, 31, 42 and 45. Mice were boosted with peptide on day 120. Melan-A tetramer levels were measured in the mice at 7-10 days following each cycle of immunization and periodically until 90 days after the second immunization cycle. (Figure 18). All seven animals showed borderline % frequencies of tetramer+ cells during and after the two immunization cycles but mounted strong responses after a peptide boost, demonstrating substantial immune memory.

### Example 18. Various combinations of antigen plus immunopotentiating adjuvant are effective for entrainment of a CTL response.

Intranodal administration of peptide is a very potent means to amplify immune responses triggered by intralymphatic administration of agents (replicative or non-replicative) comprising or in association with adjuvants such as TLRs.

Subjects (such as mice, humans, or other mammals) are entrained by intranodal infusion or injection with vectors such as plasmids, viruses, peptide plus adjuvant (CpG, dsRNA, TLR ligands), recombinant protein plus adjuvant (CpG, dsRNA, TLR ligands), killed microbes or purified antigens (e.g., cell wall components that have immunopotentiating activity) and amplified by intranodal injection of peptide without adjuvant. The immune response measured before and after boost by tetramer staining and other methods shows substantial increase in magnitude. In contrast, a boost utilizing peptide without adjuvant by other routes does not achieve the same increase of the immune response.

### Example 19. Intranodal administration of peptide is a very potent means to amplify immune responses triggered by antigen plus immunopotentiating adjuvant through any route of administration.

Subjects (such as mice, humans, or other mammals) are immunized by parenteral or mucosal administration of vectors such as plasmids, viruses, peptide plus adjuvant (CpG, dsRNA, TLR ligands), recombinant protein plus adjuvant (CpG, dsRNA, TLR ligands), killed microbes or purified antigens (*e.g*., cell wall components that have immunopotentiating activity) and amplified by intranodal injection of peptide without adjuvant. The immune response measured before and after boost by tetramer staining and other methods shows substantial increase in magnitude. In contrast, a boost utilizing peptide without adjuvant by other routes than intranodal does not achieve the same increase of the immune response.

### Example 20. Tolerance Breaking using an Entrain-and-Amplify Immunization protocol.

In order to break tolerance or restore immune responsiveness against self-antigens (such as tumor-associated antigens) subjects (such as mice, humans, or other mammals) are immunized with vectors such as plasmids, viruses, peptide plus adjuvant (CpG, dsRNA, TLR mimics), recombinant protein plus adjuvant (CpG, dsRNA, TLR mimics), killed microbes or purified antigens and boosted by intranodal injection with peptide (corresponding to a self epitope) without adjuvant. The immune response measured before and after boost by tetramer staining and other methods shows substantial increase in the magnitude of immune response ("tolerance break").

### Example 21. Clinical practice for entrain-and-amplify immunization.

Patients are diagnosed as needing treatment for a neoplastic or infectious disease using clinical and laboratory criteria; treated or not using first line therapy; and referred to evaluation for active immunotherapy. Enrollment is made based on additional criteria (antigen profiling, MHC haplotyping, immune responsiveness) depending on the nature of disease and characteristics of the therapeutic product. The treatment (Figure 19) is carried out by intralymphatic injection or infusion (bolus, programmable pump, or other means) of vector (plasmids) and protein antigens (peptides) in a precise sequence. The most preferred protocol involves repeated cycles encompassing plasmid entrainment followed by amplifying dose(s) of peptide. The frequency and continuation of such cycles can be adjusted depending on the response measured by immunological, clinical and other means. The composition to be administered can be monovalent or polyvalent, containing multiple vectors, antigens, or epitopes. Administration can be to one or multiple lymph nodes simultaneously or in staggered fashion. Patients receiving this therapy demonstrate amelioration of symptoms.

### Example 22. Clinic practice for induction of immune deviation or de-activation of pathogenic T cells.

Patients with autoimmune or inflammatory disorders are diagnosed using clinical and laboratory criteria, treated or not using first line therapy, and referred to evaluation for active immunotherapy. Enrollment is made based on additional criteria (antigen profiling, MHC haplotyping, immune responsiveness) depending on the nature of disease and characteristics of the therapeutic product. The treatment is carried out by intralymphatic injection or infusion (bolus, programmable pump or other means) of peptide devoid of T1-promoting adjuvants and/or together with immune modulators that amplify immune deviation. However, periodic bolus injections are the preferred mode for generating immune deviation by this method. Treatments with peptide can be carried weekly, biweekly or less frequently (e.g., monthly), until a desired effect on the immunity or clinical status is obtained. Such treatments can involve a single administration, or multiple closely spaced administrations as in figure 2, group 2. Maintenance therapy can be afterwards initiated, using an adjusted regimen that involves less frequent injections. The composition to be administered can be monovalent or polyvalent, containing multiple epitopes. It is preferred that the composition be free of any component that would prolong residence of peptide in the lymphatic system. Administration can be to one or multiple lymph nodes simultaneously or in staggered fashion and the response monitored by measuring T cells specific for immunizing peptides or unrelated epitopes ("epitope spreading"), in addition to pertinent clinical methods.

### Example 23. Immunogenic Compositions (e.g., Viral Vaccines)

Six groups (n=6) of HLA-A2 transgenic mice are injected with 25 ug of plasmid vector bilaterally in the inguinal lymph nodes, according to the following schedule: day 0, 3, 14 and 17. The vector encodes three A2 restricted epitopes from HIV *gag* (SLYNTVATL, VLAEAMSQV, MTNNPPIPV), two from pol (KLVGKLNWA, ILKEPVHGV) and one from env (KLTPLCVTL). Two weeks after the last cycle of entrainment, mice are injected with mixtures encompassing all these five peptides (5ug/peptide/node bilaterally three days apart). In parallel, five groups of mice are injected with individual peptides (5ug/peptide/node bilaterally three days apart). Seven days later the mice are bled and response is assessed by tetramer staining against each peptide. Afterwards, half of the mice are challenged with recombinant *Vaccinia* viruses expressing *env, gag or pol* (10³ TCID₅₀/mouse) and at 7 days, the viral titer is measured in the ovaries by using a conventional plaque assay. The other half are sacrificed, the splenocytes are stimulated with peptides for 5 days and the cytotoxic activity is measured against target cells coated with peptides. As controls, mice were injected with plasmid or peptides alone. Mice entrained with plasmid and amplified with peptides show stronger immunity against all five peptides, by tetramer staining and cytotoxicity.

More generally, in order to break tolerance, restore immune responsiveness or induce immunity against non-self antigens such as viral, bacterial, parasitic or microbial, subjects (such as mice, humans, or other mammals) are immunized with vectors such as plasmids, viruses, peptide plus adjuvant (CpG, dsRNA, TLR mimics), recombinant protein plus adjuvant (CpG, dsRNA, TLR mimics), killed microbes or purified antigens (such as cell wall components) and boosted by intranodal injection with peptide (corresponding to a self epitope) without adjuvant. The immune response measured before and after boost by tetramer staining and other methods shows substantial increase in the magnitude of immune response. Such a strategy can be used to protect against infection or treat chronic infections caused by agents such as HBV, HCV, HPV, CMV, influenza virus, HIV, HTLV, RSV, etc.

Many variations and alternative elements of the invention have been disclosed. Still further variations and alternate elements will be apparent to one of skill in the art. Various embodiments of the invention can specifically include or exclude any of these variation or elements.

### Some embodiments of the invention are disclosed in the following items.

### Items

1. A method of immunization comprising: delivering to a mammal a first composition comprising an immunogen, the immunogen comprising or encoding at least a portion of a first antigen; and administering a second composition, comprising an amplifying peptide, directly to a lymphatic system of the mammal, wherein the peptide corresponds to an epitope of said first antigen, wherein the first composition and the second composition are not the same.
2. The method of item 1 wherein the first composition comprises a nucleic acid encoding the antigen or an immunogenic fragment thereof.
3. The method of item 1 wherein the first composition comprises a nucleic acid capable of expressing the epitope in a pAPC.
4. The method of item 2 or 3 wherein the nucleic acid is delivered as a component of a protozoan, bacterium, virus, or viral vector.
5. The method of item 1 wherein the first composition comprises an immunogenic polypeptide and an immunopotentiator.
6. The method of item 5 wherein the immunopotentiator is a cytokine.
7. The method of item 5 wherein the immunopotentiator is a toll-like receptor ligand.
8. The method of item 7 wherein the adjuvant comprises an immunostimulatory sequence.
9. The method of item 7 wherein the adjuvant comprises RNA.
10. The method of item 5 wherein the immunogenic polypeptide is said amplifying peptide.
11. The method of item 5 wherein the immunogenic polypeptide is said first antigen.
12. The method of item 5 wherein the immunogenic polypeptide is delivered as a component of a protozoan, bacterium, virus, viral vector, or virus-like particle.
13. The method of item 5 wherein the adjuvant is delivered as a component of a protozoan, bacterium, virus, viral vector, or virus-like particle.
14. The method of item 1 wherein the second composition is adjuvant-free and immunopotentiator-free.
15. The method of item 1 wherein the delivering step comprises direct administration to the lymphatic system of the mammal.
16. The method of item 1 or item 15 wherein direct administration to the lymphatic system of the mammal comprises direct administration to a lymph node or lymph vessel.
17. The method of item 16 wherein direct administration is to two or more lymph nodes or lymph vessels.
18. The method of item 16 wherein the lymph node is selected from group consisting of inguinal, axillary, cervical, and tonsilar lymph nodes.
19. The method of Item 1, further comprising obtaining an effector T cell response to the first antigen.
20. The method of item 19 wherein the effector T cell response comprises production of a pro-inflammatory cytokine.
21. The method of item 20 wherein the cytokine is gamma-IFN or TNF-alpha.
22. The method of item 19 wherein the effector T cell response comprises production of a T cell chemokine.
23. The method of item 22 wherein the chemokine is RANTES or MIP-1alpha.
24. The method of item 1 wherein the epitope is a housekeeping epitope.
25. The method of item 1 wherein the epitope is an immune epitope.
26. The method of item 1 wherein the delivering step or the administering step comprises a single bolus injection.
27. The method of item 1 wherein the delivering step or the administering step comprises repeated bolus injections.
28. The method of item 1 wherein the delivering step or the administering step comprises a continuous infusion.
29. The method of item 28 wherein the infusion has a duration of between about 8 to about 7 days.
30. The method of item 1 having an interval between termination of the delivering step and beginning the administering step, wherein the interval is at least about seven days.
31. The method of item 30 wherein the interval is between about 7 and about 14 days.
32. The method of item 30 wherein the interval is over about 75 days.
33. The method of item 1 wherein the first antigen is a disease-associated antigen.
34. The method of item 33 wherein the disease-associated antigen is a tumor- associated antigen.
35. The method of item 34 wherein the disease-associated antigen is a pathogen-associated antigen.
36. The method of treating a disease comprising the method of item 35.
37. The method of item 1 wherein the first antigen is a target-associated antigen.
38. The method of item 37 wherein the target is a neoplastic cell.
39. The method of item 37 wherein the target is a pathogen-infected cell.
40. The method of item 37 wherein the pathogen infected cell is infected by a protozoa, a bacterium, a fungi, a virus, or a prion.
41. The method of item 1 wherein the effector T cell response is detected by at least one indicator selected from the group consisting of a cytokine assay, an Elispot assay, a cytotoxicity assay, a tetramer assay, a DTH-response, a clinical response, tumor shrinkage, tumor clearance, inhibition of tumor progression, decrease pathogen titre, pathogen clearance, and amelioration of a disease symptom.
42. The method of item 19 wherein the effector T cell response is a cytotoxic T cell response.
43. A method of immunization comprising: delivering to a mammal a first composition comprising a nucleic acid encoding a first antigen or an immunogenic fragment thereof; and administering a second composition, comprising a peptide, directly to the lymphatic system of the mammal, wherein the peptide corresponds to an epitope of said first antigen
44. The method of item 43 further comprising obtaining an effector T cell response to the antigen.
45. A method of augmenting an existing antigen-specific immune response comprising: administering a composition, comprising a peptide, directly to the lymphatic system of a mammal, wherein the peptide corresponds to an epitope of said antigen, and wherein said composition was not used to induce the immune response; and obtaining augmentation of an antigen-specific immune response.
46. The method of item 45 wherein the augmentation comprises sustaining the response over time.
47. The method of item 45 wherein the augmentation comprises reactivating quiescent T cells.
48. The method of item 45 wherein the augmentation comprises expanding the population of antigen-specific T cells.
49. The method of item 45 wherein said composition does not comprise an immunopotentiator.
50. A method of immunization comprising: delivering to a mammal a first composition comprising an immunogen, the immunogen comprising or encoding at least a portion of a first antigen and at least a portion of a second antigen ; and administering a second composition comprising a first peptide, and a third composition comprising a second peptide, directly to the lymphatic system of the mammal, wherein the first peptide corresponds to an epitope of said first antigen, and wherein the second peptide corresponds to an epitope of said second antigen, wherein the first composition is not the same as the second or third compositions.
51. The method of item 50 further comprising obtaining an effector T cell response to the antigen.
52. The method of item 50 wherein said second and third compositions each comprise the first and the second peptides.
53. A method of generating an antigen-specific tolerogenic or regulatory immune response comprising: periodically administering a composition, comprising an adjuvant-free peptide, directly to the lymphatic system of a mammal, wherein the peptide corresponds to an epitope of said antigen, and wherein the mammal is epitopically naive.
54. The method of item 53, further comprising obtaining a tolerogenic or regulatory T cell immune response.
55. The method of item 53, wherein the immune response assists in treating an inflammatory disorder.
56. The method of item 55 wherein the inflammatory disorder arises from a class II MHC-restricted immune response.
57. The method of item 53 wherein the immune response comprises production of an immunosuppressive cytokine.
58. The method of item 57 wherein the cytokine is IL-5, IL-10, or TGB-beta.
59. A method of immunization comprising: administering a series of immunogenic doses directly into the lymphatic system of a mammal wherein the series comprises at least 1 entraining dose and at least 1 amplifying dose, and wherein the entraining dose comprises a nucleic acid encoding an immunogen and wherein the amplifying dose is free of any virus, viral vector, or replication-competent vector.
60. The method of item 59 comprising 1-6 entraining doses.
61. The method of item 59 comprising administering a plurality of entraining doses, wherein said doses are administered over a course of one to about seven days.
62. The method of item 59 wherein entraining doses, amplifying doses, or entraining and amplifying doses are delivered in multiple pairs of injections, wherein a first member of a pair is administered within about 4 days of a second member of the pair, and wherein an interval between first members of different pairs is at least about 14 days.
63. The method of item 62 wherein an interval between a last entraining dose and a first amplifying dose is between about 7 and about 100 days.
64. The method of item 59, further comprising obtaining an antigen-specific immune response.
65. A set of immunogenic compositions for inducing an immune response in a mammal comprising 1-6 entraining doses and at least one amplifying dose, wherein the entraining doses comprise a nucleic acid encoding an immunogen, and wherein the amplifying dose comprises a peptide epitope, and wherein the epitope is presented by pAPC expressing the nucleic acid.
66. The set of item 65 wherein one dose further comprises an adjuvant.
67. The set of item 66 wherein the adjuvant is RNA.
68. The set of item 65 wherein the entraining and amplifying doses are in a carrier suitable for direct administration to the lymphatic system.
69. The set of item 68 wherein the entraining and amplifying doses are in a carrier suitable for direct administration to a lymph node.
70. The set of item 65 wherein the nucleic acid is a plasmid.
71. The set of item 65 wherein the epitope is a class I HLA epitope.
72. The set of item 71 wherein the class I HLA is selected from Tables 1-4.
73. The set of item 72 wherein the HLA is HLA-A2.
74. The set of item 65 wherein the immunogen comprises an epitope array.
75. The set of item 74 wherein the epitope array comprises a liberation sequence.
76. The set of item 65 where in the immunogen consists essentially of a target- associated antigen.
77. The set of item 76 wherein the target-associated antigen is a tumor- associated antigen.
78. The set of item 76 wherein the target-associated antigen is a microbial antigen.
79. The set of item 65 wherein the immunogen comprises a fragment of a target-associated antigen that comprises an epitope cluster.
80. A set of immunogenic compositions for inducing a class I MHC-restricted immune response in a mammal comprising 1-6 entraining doses and at least one amplifying dose, wherein the entraining doses comprise an immunogen or a nucleic acid encoding an immunogen and an immunopotentiator, and wherein the amplifying dose comprises a peptide epitope, and wherein the epitope is presented by pAPC.
81. The set of item 80 wherein the nucleic acid encoding the immunogen further comprises an immunostimulatory sequence with serves as the immunopotentiating agent.
82. The set of item 80 wherein the immunogen is a virus or replication- competent vector that comprises or induces an immunopotentiating agent.
83. The set of item 80 wherein the immunogen is a bacterium, bacterial lysate, or purified cell wall component wherein the bacterial cell wall component serves as the immunopotentiating agent.
84. The set of item 80 wherein the immunopotentiating agent is selected from the group consisting of a TLR ligand, an immunostimulatory sequence, a CpG-containing DNA, a dsRNA, an endocytic-Pattern Recognition Receptor (PRR) ligand, an LPS, a quillaja saponin, tucaresol, and a pro-inflammatory cytokine.

## Claims

1. A set of immunogenic compositions for inducing a class I MHC-restricted immune response in a mammal comprising 1-6 entraining doses and at least one amplifying dose, wherein the entraining doses comprise an immunopotentiator and a nucleic acid encoding an immunogen, and wherein the amplifying dose comprises a peptide epitope, and wherein the epitope is presented by pAPC.

2. The set of claim 1 wherein the nucleic acid encoding the immunogen further comprises an immunostimulatory sequence with serves as the immunopotentiating agent.

3. The set of claim 1 wherein the immunogen is a virus or replication- competent vector that comprises or induces an immunopotentiating agent.

4. The set of claim 1 wherein the immunogen is a bacterium, bacterial lysate, or purified cell wall component wherein the bacterial cell wall component serves as the immunopotentiating agent.

5. The set of claim 1 wherein the immunopotentiating agent is selected from the group consisting of a TLR ligand, an immunostimulatory sequence, a CpG-containing DNA, a dsRNA, an endocytic-Pattern Recognition Receptor (PRR) ligand, an LPS, a quillaja saponin, tucaresol, and a pro-inflammatory cytokine.

6. The set of claim 1, wherein said set is for use in the treatment of a neoplastic or infectious disease.

7. The set of claim 1, wherein said immunogenic compositions comprise an entraining and an amplifying composition, wherein the entraining composition comprises a non-replicating plasmid encoding an immunogen and wherein the amplifying composition comprises an amplifying peptide.

8. The set of claim 1, wherein said immunogenic compositions are polyvalent.

9. The set of claim 1, wherein said immunogenic compositions are administered by injection or infusion into one or more lymph nodes.
